(19) Europäisches Patentamt
European Patent Office
Office européen des brevets

(11) EP 1 743 643 A1

(12) EUROPEAN PATENT APPLICATION

(43) Date of publication:
**17.01.2007 Bulletin 2007/03**

(51) Int Cl.:
***A61K 31/4155*** *(2006.01)* ***A61K 31/496*** *(2006.01)*
***A61K 31/454*** *(2006.01)* ***A61K 9/51*** *(2006.01)*

(21) Application number: **05384027.8**

(22) Date of filing: **15.07.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **LABORATORIOS DEL DR. ESTEVE, S.A.**
**08041 Barcelona (ES)**

(72) Inventor: **Buschmann, Helmut Heinrich**
**08960 Sant Just Desvern (Barcelona) (ES)**

(54) **New formulations of substituted pyrazoline compounds**

(57) The present invention relates to new formulations of substituted pyrazoline compounds their derivatives as well as their physiologically acceptable salts with nanoparticles.

EP 1 743 643 A1

## Description

The present invention relates to new formulations of substituted pyrazoline compounds their derivatives as well as their physiologically acceptable salts with nanoparticles.

## Background of the invention

Drug delivery is one of the most critical points in the development of a drug. Many therapeutic compounds demonstrate good in vitro, in vivo and pharmacological properties but finally fail to overcome the last hurdle: To be on the correct site of action. As a result undesired side effects may occur because of too high drug doses. By utilizing alternative, targeting drug delivery systems as are for example nanoparticles, drugs can be transported directly to their site of action by crossing natural biological barriers as for instance the blood-brain barrier or the skin. This means a significant advantage over traditional formulations since less active ingredient is needed to have the same therapeutic effect. Moreover, the safety profile of a given drug with respect to adverse side effects improves dramatically.

Cannabinoids are compounds, which are derived from the cannabis sativa plant which is commonly known as marijuana. The most active chemical compound of the naturally occurring cannabinoids is tetrahydrocannabinol (THC), particularly $\Delta^9$-THC.

These naturally occuring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated $CB_1$ and $CB_2$ are involved in a variety of physiological or pathophysiological processes in humans and animals, e.g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992.

Therefore, compounds, which have a high binding affinity for these cannabinoid receptors and which are suitable for modulating these receptors are useful in the prevention and/or treatment of cannabinoid-receptor related disorders.

It has been found that these compounds have a high affinity for cannabinoid receptors, particularly for the $CB_1$-receptor, and that they act as modulators e.g. antagonists, inverse agonists or agonists on these receptors. They are therefore suitable for the prophylaxis and/or treatment of various disorders related to the central nervous system, the immune system, the cardiovascular system, the endocrinous system, the respiratory system, the gastrointestinal tract or reproduction in humans and/or animals, preferably humans including infants, children and grown-ups.

Surprisingy it was found that pharmaceutical formulations containing these compounds associated with nanoparticles are highly advantageous over traditional formulations with respect to drug delivery to the site of action.

Therefore, the present invention relates to new pharmaceutical formulations comprising substituted pyrazoline compounds associated with nanoparticles.

Nanoparticles represent an innovative drug delivery system (For review see:

Pharmazie; 2004; 59: 5-9). These particles are solid or semi-solid colloidal particles ranging from 1 to 1000 nm in diameter, consisting of macromolecular material which can optionally be biodegradable.

The active principle, optionally in dissolved form may be entrapped or encapsulated in the particles, or attached to the surface of the particles, for example via adsorption. Nanoparticles can among other things be used for targeted delivery of drugs (as exemplified by disclosure in US.6,117,454), to sustain drug effects in target tissue (J Pharm Pharmaceut Sci, 2000, 3:234-258), to improve oral bioavailability, to solubilize drugs for intravascular delivery or to improve the stability of therapeutic agents. Another important aspect of nanoparticle technology is the topical delivery of drugs and vaccines through the skin (Expert Rev Vaccines, 2003, 2:753-761; Adv Drug Delivery Rev, 2002, 54: S131-155).

As a specific example, drugs used to treat the nervous system in mammals, in particular in human patients, have to be able to cross the blood brain barrier (bbb). The term "blood brain barrier" (bbb) as used herein refers to the bbb in the narrower sense, i. e. in the sense this term is used usually by a person skilled in the medical field, as well as to the blood spinal barrier and blood retina barrier. Unfortunately, many drugs do not pass the bbb efficiently or not at all and are only effective when given directly into the brain. The blood brain barrier (bbb), which consists of the endothelium of the brain vessels, the basal membrane and neuroglial cells, acts to limit transport of substances into the brain. Sometimes the structure of the bbb is subdivided into two components: the endothelial or capillary barrier and the ependymal barrier (Banks, W. A., Kastin, A. J., Barrera, Delivering peptides to the central nervous system: Dilemmas and strategies, Pharm. Res. 8 (1991), 1345-1350). The nature of the substance penetration through the bbb has not yet been determined but it is known that many of the regulators of brain function such as cytokines, transferrin, enkephalines, endorphines can pass through the bbb from the vessels into the. However, many substances which can affect the central

nervous system (CNS) such as adenosin, [beta]-endorphins, synthetic analogues of endogenous peptides (Houghten et al. 1980, Levin et al 1987, Sakane et al. 1989) as well as some excitatory and inhibitor amino acids and trophic factors, penetrate poorly or not at all through the bbb. At present, drugs with no bbb penetration or poor bbb penetration can only be given by direct CNS infusion. Thus, many potentially potent drugs are not useful clinically due to their inability to pass the bbb.

The term "nanoparticles" as mentioned in this invention refers to particles of a size from 1 to 1000 nm in diameter and includes but is not restricted to any type of nanoparticles such as solid lipid nanoparticles (SLN), a nanosuspension, nanocrystals, polymeric nanoparticles, microparticles, a nanoemulsion, nanostructured lipid carriers (NLC), micelles, liquid crystals, liposomes, or nanocapsules. Said nanoparticles are preferably solid, semi-solid, crystalline, amorphous or noncrystalline and may be stabilized by suitable additives such as polymers and/or lipids and are at optionally at least partially biodegradable.

As used herein, the terms "biodegradable"or"bioerodible" refer to components of nanoparticles that can for example degrade into low molecular weight compounds, which are known to be involved normally in metabolic pathways. The terms also include nanoparticle systems which can be broken down in the biological milieu so that the integrity of the system, and in some cases its components such as macromolecules is affected and gives fragments or other degradation by-products which can be transported.

The term "associated" according to the present invention means that a substituted pyrazoline compound as defined herein, is bonded to the nanoparticles via chemical or physical interaction such as covalent bonding, van der Waal's bonding, hydrogen bonding, ionic interaction, or complexation. Optionally, said pyrazoline compounds may be absorbed by a nanoparticle, adsorbed to a nanoparticle, may be inside a nanoparticle, or may be enclosing a nanoparticle.

Thus, an aspect of the invention relates to a pharmaceutical formulation comprising

(A) at least one substituted pyrazoline compound of general formula I

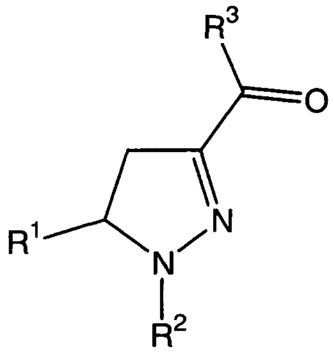

**I**

wherein

$R^1$ represents an optionally at least mono-substituted phenyl group;

$R^2$ represents an optionally at least mono-substituted phenyl group;

$R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or $R^3$ represents an optionally at least mono-substituted aryl or heteroaryl group, which may be condensed with an optionally at least mono-substituted mono- or polycyclic ring system, or $R^3$ represents an $-NR^4R^5$-moiety,

$R^4$ and $R^5$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted aryl or heteroaryl group, which may be condensed with an optionally at least mono-substituted mono- or polycyclic ring system and/or bonded via a linear or branched alkylene group; an $-SO_2-R^6$-moiety; or an $-NR^7R^8$-moiety, with the proviso that $R^4$ and $R^5$ do not identically represent hydrogen;

$R^6$ represents a linear or branched, saturated or unsaturated, optionally at least mono-substituted aliphatic group; a saturated or unsaturated, optionally at least mono-substituted, optionally at least one heteroatom as ring member containing cycloaliphatic group, which may be condensed with a mono- or polycyclic ring-system; or an optionally at least mono-substituted aryl or heteroaryl group, which may be condensed with a mono- or polycyclic ring system and/or bonded via a linear or branched alkylene group;

$R^7$ and $R^8$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted aryl or heteroaryl group, which may be condensed with an optionally at least mono-substituted mono- or polycyclic ring system and/or bonded via a linear or branched alkylene group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and/or
at least one substituted pyrazoline compound of general formula I',

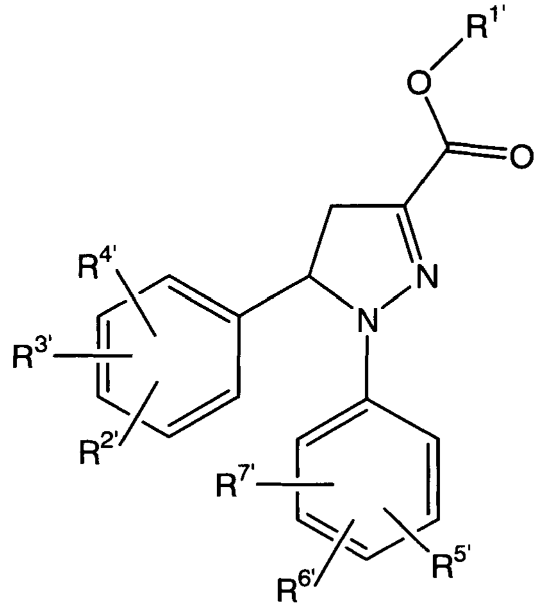

**I'**

wherein

$R^{1'}$ represents hydrogen or a linear or branched $C_{1-4}$-alkyl group,

$R^{2'}$, $R^{3'}$ and $R^{4'}$ independently of each other represent hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{8'}$, SH, $SR^{8'}$. $SOR^{8'}$, $SO_2R^{8'}$, $NH_2$, $NHR^{8'}$, $NR^{8'}R^{9'}$, -(C=O)-$NH_2$, -(C=O)-$NHR^{8'}$ or -(C=O)-$NR^{8'}R^{9'}$ whereby $R^{8'}$ and $R^{9'}$ for each substituent independently represent linear or branched
$C_{1-6}$ alkyl,

$R^{5'}$ and $R^{6'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10'}$, SH, $SR^{10'}$, $SOR^{10'}$, $NH_2$, $NHR^{10'}$, $NR^{10'}R^{11'}$, -(C=O)-$NH_2$, -(C=O)-$NHR^{10'}$ and -(C=O)-$NR^{10'}R^{11'}$, whereby $R^{10'}$ and optionally $R^{11'}$ for

each substituent independently represent linear or branched $C_{1-6}$ alkyl;

$R^{7'}$ represents hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10'}$, SH, $SR^{10'}$, $SOR^{10'}$, $NH_2$, $NHR^{10'}$, $NR^{10'}R^{11'}$, -(C=O)-$NH_2$, -(C=O)-$NHR^{10'}$ and -(C=O)-$NR^{10'}R^{11'}$, whereby $R^{10'}$ and optionally $R^{11'}$ for each substituent independently represent linear or branched $C_{1-6}$ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and

(B) nanoparticles,

wherein
component (A) is associated with component (B).

With respect to compounds of general formula I,
a mono- or polycyclic ring-system according to the present invention means a mono- or polycyclic hydrocarbon ring-system that may be saturated, unsaturated or aromatic. If the ring system is polycyclic, each of its different rings may show a different degree of saturation, i.e. it may be saturated, unsaturated or aromatic. Optionally each of the rings of the mono- or polycyclic ring system may contain one or more, e.g. 1, 2 or 3, heteroatoms as ring members, which may be identical or different and which can preferably be selected from the group consisting of N, O, S and P, more preferably be selected from the group consisting of N, O and S. Preferably the polycyclic ring-system may comprise two rings that are condensed. The rings of the mono- or polycyclic ring-sytem are preferably 5- or 6-membered.

The term "condensed" according to the present invention means that a ring or ring-system is attached to another ring or ring-system, whereby the terms "annulated" or "annelated" are also used by those skilled in the art to designate this kind of attachment.

If one or more of the residues $R^3$-$R^8$ represents or comprises a saturated or unsaturated, optionally at least one heteroatom as ring member containing cycloaliphatic group, which is substituted by one or more, e.g. 1, 2, 3 or 4, substituents, unless defined otherwise, each of the substituents may be independently selected from the group consisting of hydroxy, fluorine, chlorine, bromine, branched or unbranched $C_{1-6}$-alkoxy, branched or unbranched $C_{1-6}$-alkyl, branched or unbranched $C_{1-4}$-perfluoroalkoxy, branched or unbranched $C_{1-4}$-perfluoroalkyl, oxo, amino, carboxy, amido, cyano, nitro, -$SO_2NH_2$, -CO-$C_{1-4}$-alkyl, -SO-$C_{1-4}$-alkyl, -$SO_2$-$C_{1-4}$-alkyl, -NH-$SO_2$-$C_{1-4}$-alkyl , wherein the $C_{1-4}$-alkyl may in each case be branched or unbranched, and a phenyl group, more preferably be selected from the group consisting of hydroxy, F, Cl, Br, methyl, ethyl, methoxy, ethoxy, oxo, $CF_3$ and a phenyl group.

If one or more of the residues $R^3$-$R^8$ represents or comprises a cycloaliphatic group, which contains one or more heteroatoms as ring members, unless defined otherwise, each of these heteroatoms may preferably be selected from the group consisting of of N, O and S. Preferably a cycloaliphatic group may contain 1, 2 or 3 heteratoms independently selected from the group consisting of N, O and S as ring members.

Suitable saturated or unsaturated, optionally at least one heteroatom as ring member containing, optionally at least mono-substituted cycloaliphatic groups may preferably be selected from the group consisting of Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, Pyrrolidinyl, Piperidinyl, Piperazinyl, homo-Piperazinyl and Morpholinyl.

If one or more of the residues $R^3$-$R^8$ comprises a mono- or polycyclic ring system, which is substituted by one or more, e.g. 1, 2, 3, 4 or 5 substituents, unless defined otherwise, each of the substituents may be independently selected from the group consisting of hydroxy, fluorine, chlorine, bromine, branched or unbranched $C_{1-6}$-alkoxy, branched or unbranched $C_{1-6}$-alkyl, branched or unbranched $C_{1-4}$-perfluoroalkoxy, branched or unbranched $C_{1-4}$-perfluoroalkyl, amino, carboxy, oxo, amido, cyano, nitro, -$SO_2NH_2$, -CO-$C_{1-4}$-alkyl, -SO-$C_{1-4}$-alkyl, -$SO_2$-$C_{1-4}$-alkyl, -NH-$SO_2$-$C_{1-4}$-alkyl , wherein the $C_{1-4}$-alkyl may in each case be branched or unbranched, and a phenyl group, more preferably be selected from the group consisting of hydroxy, F, Cl, Br, methyl, ethyl, methoxy, ethoxy, $CF_3$, oxo and a phenyl group.

If one or more of the residues $R^1$-$R^8$ represents or comprises an aryl group, including a phenyl group, which is substituted by one or more, e.g. 1, 2, 3, 4 or 5 substituents, unless defined otherwise, each of the substituents may be independently selected from the group consisting of a halogen atom (e.g. F, Cl, Br, I), a linear or branched $C_{1-6}$-alkyl group, a linear or branched $C_{1-6}$ alcoxy group, a formyl group, a hydroxy group, a trifluoromethyl group, a trifluoromethoxy group, a -CO-$C_{1-6}$-alkyl group, a cyano group, a nitro group, a carboxy group, a -CO-O-$C_{1-6}$-alkyl group, a -CO-$NR^AR^B$-moiety, a -CO-NH-$NR^CR^D$-moiety, an —SH, an -S-$C_{1-6}$-alkyl group, an -SO-$C_{1-6}$-alkyl group, an -$SO_2$-$C_{1-6}$-alkyl group, a -$C_{1-6}$-alkylene-S-$C_{1-6}$-alkyl group, a -$C_{1-6}$-alkylene-SO-$C_{1-6}$-alkyl group, a -$C_{1-6}$-alkylene-$SO_2$-$C_{1-6}$-alkyl group, an —$NH_2$-moiety, an NHR'-moiety or an NR'R''-moiety, wherein R' and R'' independently represent a linear or branched $C_{1-6}$-alkyl group, a $C_{1-6}$-alkyl group substituted by one or more hydroxy groups and a —$C_{1-6}$-alkylene-$NR^ER^F$ group,

whereby $R^A$, $R^B$, identical or different, represent hydrogen or a $C_{1-6}$-alkyl group, or $R^A$ and $R^B$ together with the bridging nitrogen atom form a saturated, mono- or bicyclic, 3-10 membered heterocyclic ring system, which may be at least mono-substituted by one or more, identical or different, $C_{1-6}$ alkyl groups and/or which may contain at least one further heteroatom selected from the group consisting of nitrogen, oxygen and sulphur as a ring member,
$R^C$, $R^D$, identical or different, represent a hydrogen atom, a $C_{1-6}$-alkyl group, a -CO-O-$C_{1-6}$-alkyl group, a $C_{3-8}$-cycloalkyl group, a $C_{1-6}$-alkylene-$C_{3-8}$-cycloalkyl group, $C_{1-6}$-alkylene-O-$C_{1-6}$-alkyl group or a $C_{1-6}$-alkyl group substituted with one or more hydroxy groups, or $R^C$, $R^D$ together with the bridging nitrogen atom form a saturated, mono- or bicyclic, 3-10 membered heterocyclic ring system, which may be at least mono-substituted by one or more substituents independently selected from the group consisting of $C_{1-6}$ alkyl group, a -CO-$C_{1-6}$-alkyl group, a —CO-O- $C_{1-6}$-alkyl group, a -CO-NH-$C_{1-6}$-alkyl group, a -CS-NH-$C_{1-6}$-alkyl group, an oxo group, a $C_{1-6}$-alkyl group substituted with one or more hydroxy groups, a $C_{1-6}$-alkylene-O-$C_{1-6}$-alkyl group and a -CO-$NH_2$ group and/or which may contain at least one further heteroatom selected from the group consisting of nitrogen, oxygen and sulphur as a ring member, and
wherein $R^E$, $R^F$, identical or different, represent hydrogen or a $C_{1-6}$-alkyl group, or $R^E$ and $R^F$ together with the bridging nitrogen atom form a saturated, mono- or bicyclic, 3-10 membered heterocyclic ring system, which may be at least mono-substituted by one or more, identical or different $C_{1-6}$ alkyl groups and/or which may contain at least one further heteroatom selected from the group consisting of nitrogen, oxygen and sulphur as a ring member.

Preferred aryl groups, which may optionally be at least mono-substituted, are phenyl and naphthyl.

If one or more of the residues $R^3$-$R^8$ represents or comprises a heteroaryl group, which is substituted by one or more, e.g. 1, 2, 3, 4 or 5 substituents, unless defined otherwise, each of the substituents may be independently selected from the group consisting of a halogen atom (e.g. F, Cl, Br, I), a linear or branched $C_{1-6}$-alkyl group, a linear or branched $C_{1-6}$alcoxy group, a formyl group, a hydroxy group, a trifluoromethyl group, a trifluoromethoxy group, a -CO-$C_{1-6}$-alkyl group, a cyano group, a carboxy group, a -CO-O-$C_{1-6}$-alkyl group, a -CO-$NR^AR^B$- moiety, a -CO-NH-$NR^CR^D$-moiety, an -S-$C_{1-6}$-alkyl group, an -SO-$C_{1-6}$-alkyl group, an -$SO_2$-$C_{1-6}$-alkyl group, a -$C_{1-6}$-alkylene-S-$C_{1-6}$-alkyl group, a -$C_{1-6}$-alkylene-SO-$C_{1-6}$-alkyl group, a -$C_{1-6}$-alkylene-$SO_2$-$C_{1-6}$-alkyl group, a $C_{1-6}$-alkyl group substituted by one or more hydroxy groups and a-$C_{1-6}$-alkylene-$NR^ER^F$ group,
whereby $R^A$, $R^B$, identical or different, represent hydrogen or a $C_{1-6}$-alkyl group, or $R^A$ and $R^B$ together with the bridging nitrogen atom form a saturated, mono- or bicyclic, 3-10 membered heterocyclic ring system, which may be at least mono-substituted by one or more, identical or different, $C_{1-6}$ alkyl groups and/or which may contain at least one further heteroatom selected from the group consisting of nitrogen, oxygen and sulphur as a ring member,
$R^C$, $R^D$, identical or different, represent a hydrogen atom, a $C_{1-6}$-alkyl group, a -CO-O-$C_{1-6}$-alkyl group, a $C_{3-8}$-cycloalkyl group, a $C_{1-6}$-alkylene-$C_{3-8}$-cycloalkyl group, $C_{1-6}$-alkylene-O-$C_{1-6}$-alkyl group or a $C_{1-6}$-alkyl group substituted with one or more hydroxy groups, or $R^C$, $R^D$ together with the bridging nitrogen atom form a saturated, mono- or bicyclic, 3-10 membered heterocyclic ring system, which may be at least mono-substituted by one or more substituents independently selected from the group consisting of $C_{1-6}$alkyl group, a -CO-$C_{1-6}$-alkyl group, a -CO-O-$C_{1-6}$-alkyl group, a -CO-NH-$C_{1-6}$-alkyl group, a -CS-NH- $C_{1-6}$-alkyl group, an oxo group, a $C_{1-6}$-alkyl group substituted with one or more hydroxy groups, a $C_{1-6}$-alkylene-O-$C_{1-6}$-alkyl group and a -CO-$NH_2$ group and/or which may contain at least one further heteroatom selected from the group consisting of nitrogen, oxygen and sulphur as a ring member, and
wherein $R^E$, $R^F$, identical or different, represent hydrogen or a $C_{1-6}$-alkyl group, or $R^E$ and $R^F$ together with the bridging nitrogen atom form a saturated, mono- or bicyclic, 3-10 membered heterocyclic ring system, which may be at least mono-substituted by one or more, identical or different $C_{1-6}$ alkyl groups and/or which may contain at least one further heteroatom selected from the group consisting of nitrogen, oxygen and sulphur as a ring member,

The heteroatoms, which are present as ring members in the heteroaryl radical, may, unless defined otherwise, independently be selected from the group consisting of nitrogen, oxygen and sulphur. Preferably a heteroaryl radical may comprise 1, 2 or 3 heteroatoms independently selected from the group consisting of N, O and S as ring members.

Suitable heteroaryl groups, which may optionally be at least mono-substituted, may preferably be selected from the group consisting of thienyl, furyl, pyrrolyl, pyridinyl, imidazolyl, pyrimidinyl, pyrazinyl, indolyl, chinolinyl, isochinolinyl, benzo[1,2,5]-thiodiazolyl, benzo[b]thiophenyl, benzo[b]furanyl, imidazo[2,1-b]thiazolyl, triazolyl, and pyrazolyl, more preferably be selected from the group consisting of thienyl-, benzo[1,2,5]-thiodiazolyl, benzo[b]thiophenyl, imidazo [2,1-b]thiazolyl, triazolyl and pyrazolyl.

If one or more of the residues $R^4$-$R^3$ represents or comprises a linear or branched, saturated or unsaturated aliphatic group such as an alkyl group, which is substituted by one or more, e.g. 1, 2, 3, 4 or 5 substituents, unless defined otherwise, each of the substituents may be independently selected from the group consisting of hydroxy, fluorine, chlorine, bromine, branched or unbranched $C_{1-4}$-alkoxy, branched or unbranched $C_{1-4}$-perfluoroalkoxy, branched or unbranched $C_{1-4}$-perfluoroalkyl, amino, carboxy, amido, cyano, nitro, -$SO_2NH_2$, -CO-$C_{1-4}$-alkyl, -O-$C_{1-4}$-alkyl, -$SO_2$-$C_{1-4}$-alkyl, -NH-$SO_2$-$C_{1-4}$-alkyl , wherein the $C_{1-4}$-alkyl may in each case be branched or unbranched, and a phenyl group, more preferably be selected from the group consisting of hydroxy, F, Cl, Br, methoxy, ethoxy, $CF_3$ and a phenyl group.

Preferred linear or branched, saturated or unsaturated aliphatic groups, which may be substituted by one or

more substituents, may preferably be selected from the group consisting of methyl, ethyl, n-propyl, isopropyl, n-butyl, iso-butyl, sec-butyl, tert-butyl, n-pentyl, n-hexyl, n-heptyl, n-octyl, n-nonyl, n-decyl, vinyl, ethinyl, propenyl, propinyl, butenyl and butinyl.

If any of the residues $R^4$-$R^8$ represents or comprises a linear or branched alkylene group, said alkylene group may preferably be selected from the group consisting of -methylene -($CH_2$)-, ethylene -($CH_2$-$CH_2$)-, n-propylene -($CH_2$-$CH_2$-$CH_2$)- or isopropylene -(-C($CH_3$)$_2$)-.

In another preferred aspect of the present invention the following proviso (disclaimers) applies for compounds of general formula I:

that $R^4$ and $R^5$ do not both represent a hydrogen atom, and that if one of the residues $R^4$ and $R^5$ represents a hydrogen atom or a linear or branched, saturated or unsaturated, substituted or unsubstituted aliphatic group, the other one of these residues $R^4$ and $R^5$ does not represent a substituted or unsubstituted pyridyl group, a substituted or unsubstituted pyrimidyl group, a substituted or unsubstituted pyridazyl group, a substituted or unsubstituted pyrazinyl group, a substituted or unsubstituted thienyl group, a substituted or unsubstituted benzyl group, a substituted or unsubstituted phenethyl group, a substituted or unsubstituted phenyl group, a substituted or unsubstituted phenyl group, which is condensed (attached) to at least one, optionally substituted ring or ringsystem, an -NH-phenyl-moiety, wherein the phenyl group may be at least mono-substituted, an unsubstituted or substituted thiazole group, or an unsubstituted or substituted [1,3,4]thiadiazole group.

In a preferred an aspect of the invention relates to a pharmaceutical formulation comprising

(A) at least one substituted pyrazoline compound selected from the group consisting of
N-piperidinyl-5-(4-chloro-phenyl)-1-(2,4-dichlorophenyl)-4,5-dihydro-1H-pyrazole-3-carboxamide,
(Rac-)N-piperidinyl-5-(4-chloro-phenyl)-1-(2,4-dichlorophenyl)-4,5-dihydro-1 H-pyrazole-3-carboxamide,
(S-)N-piperidinyl-5-(4-chloro-phenyl)-1-(2,4-dichlorophenyl)-4,5-dihydro-1H-pyrazole-3-carboxamide,
(R-)N-piperidinyl-5-(4-chloro-phenyl)-1-(2,4-dichlorophenyl)-4,5-dihydro-1H-pyrazole-3-carboxamide,
5-(4-Chloro-phenyl)-1-(2,4-dichloro-phenyl)-4,5-dihydro-1H-pyrazole-3-carboxylic acid-[1,2,4]-triazole-4-yl-amide,
5-(4-Chloro-phenyl)-1-(2,4-dichloro-phenyl)-4,5-dihydro-1H-pyrazole-3-carboxylic acid-(4-methyl-piperazin-1-yl)-amide,
5-(4-Chloro-phenyl)-1-(2,4-dichloro-phenyl)-4,5-dihydro-1H-pyrazole-3-carboxylic acid diethylamide,
[5-(4-Chloro-phenyl)-1-(2,4-dichloro-phenyl)-4,5-dihydro-1H-pyrazole-3-yl]-piperidine-1-yl-methanone,
N-[5-(4-Chloro-phenyl)-1-(2,4-dichlorophenyl)-4,5-dihydro-1H-pyrazole-3-carbonyl]-4-methylphenylsufonamide,
optionally in the form of a corresponding N-oxide, or a corresponding salt, or a corresponding solvate and
(B) nanoparticles,
wherein
component (A) is associated with component (B).
In another aspect of the invention, the invention relates to a pharmaceutical formulation comprising component (A) being at least one substituted pyrazoline compounds of the general formula I'

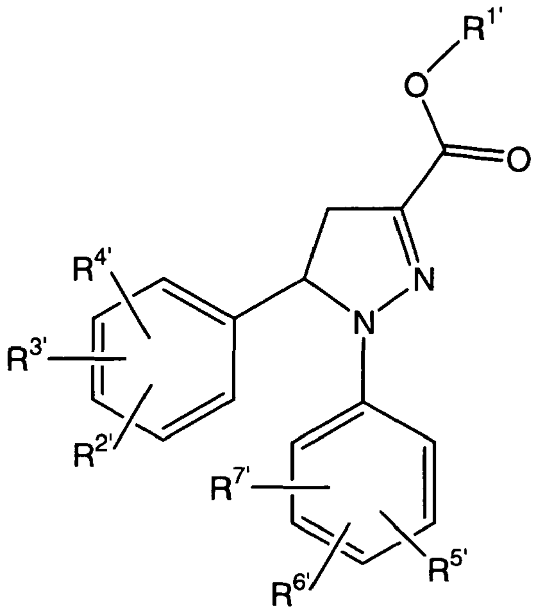

I'

wherein

$R^{1'}$ represents hydrogen or a linear or branched $C_{1-4}$-alkyl group,

$R^{2'}$, $R^{3'}$ and $R^{4'}$ independently of each other represent hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{8'}$, SH, $SR^{8'}$, $SOR^{8'}$, $SO_2R^{8'}$, $NH_2$, $NHR^{8'}$, $NR^{8'}R^{9'}$, -(C=O)-$NH_2$, -(C=O)-$NHR^{8'}$ or -(C=O)-$NR^{8'}R^{9'}$ whereby $R^{8'}$ and $R^{9'}$ for each substituent independently represent linear or branched

$C_{1-6}$ alkyl,

$R^{5'}$ and $R^{6'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10'}$, SH, $SR^{10'}$, $SOR^{10'}$, $NH_2$, $NHR^{10'}$, $NR^{10'}R^{11'}$, -(C=O)-$NH_2$, -(C=O)-$NHR^{10'}$ and -(C=O)-$NR^{10'}R^{11'}$, whereby $R^{10'}$ and optionally $R^{11'}$ for each substituent independently represent linear or branched $C_{1-6}$ alkyl;

$R^{7'}$ represents hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10'}$, SH, $SR^{10'}$, $SOR^{10'}$, $NH_2$, $NHR^{10'}$, $NR^{10'}R^{11'}$, -(C=O)-$NH_2$, -(C=O)-$NHR^{10'}$ and -(C=O)-$NR^{10'}R^{11'}$, whereby $R^{10'}$ and optionally $R^{11'}$ for each substituent independently represent linear or branched $C_{1-6}$ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and

(B) nanoparticles,

wherein component (A) is associated with component (B).

Also, preferably the pharmaceutical formulation according to the present invention comprises one or more substituted pyrazoline compounds of general formula I' given above, wherein at least one of $R^{2'}$, $R^{3'}$ or $R^{4'}$ represents

hydrogen, while at least one of $R^{2'}$, $R^{3'}$ or $R^{4'}$ is different from hydrogen.

Also, preferably the pharmaceutical formulation according to the present invention comprises one or more substituted pyrazoline compounds of general formula I' given above, wherein $R^{7'}$ represents hydrogen.

Also, preferably the pharmaceutical formulation according to the present invention comprises one or more substituted pyrazoline compounds of general formula I' given above, wherein $R^{2'}$, $R^{3'}$ and $R^{4'}$ independently of each other represent hydrogen, a linear or branched $C_{1-6}$-alkyl group, a halogen atom, or $CF_3$, preferably $R^{2'}$, $R^{3'}$ and $R^{4'}$ independently of each other represent hydrogen, methyl, ethyl, F, Cl, Br and $CF_3$.

Also, preferably the pharmaceutical formulation according to the present invention comprises one or more substituted pyrazoline compounds of general formula I' given above, wherein $R^{5'}$ and $R^{6'}$ independently of each other represent a linear or branched $C_{1-6}$-alkyl group, a halogen atom, or $CF_3$, preferably $R^{5'}$ and $R^{6'}$ independently of each other represent methyl, ethyl, F, Cl, Br and $CF_3$.

Also, preferably the pharmaceutical formulation according to the present invention comprises one or more substituted pyrazoline compounds of general formula I' given above, wherein $R^{2'}$ represents a chlorine atom in the 4-position of the phenyl ring, while $R^{3'}$ and $R^{4'}$ represent hydrogen.

Also, preferably the pharmaceutical formulation according to the present invention comprises one or more substituted pyrazoline compounds of general formula I' given above, wherein $R^{5'}$ and $R^{6'}$ each represent a chlorine atoms in the 2- and 4-position of the phenyl ring, while $R^{7'}$ represents hydrogen.

Also, preferably the pharmaceutical formulation according to the present invention comprises one or more substituted pyrazoline compounds of general formula I' given above, wherein $R^{1'}$ represents hydrogen, methyl or ethyl, preferably hydrogen.

Also, preferably the pharmaceutical formulation according to the present invention comprises one or more substituted pyrazoline compounds of general formula I' given above, wherein the compounds of general formula I' are represented by the

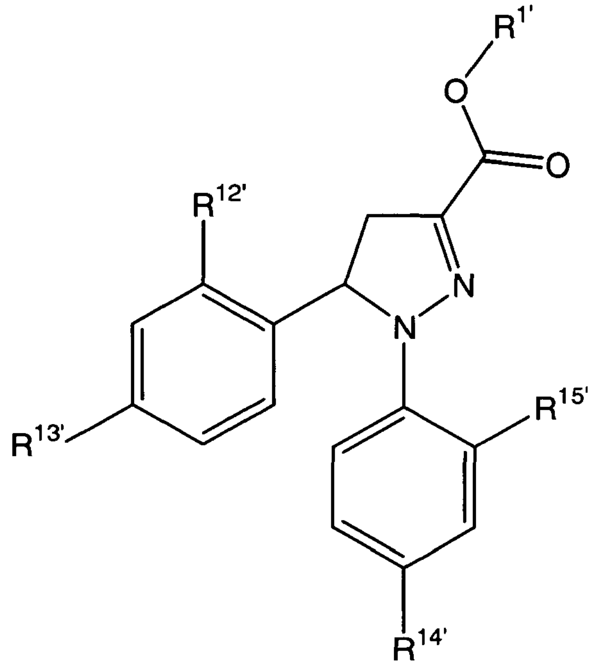

II

following structure II:
wherein

$R^{1'}$ represents hydrogen or a linear or branched $C_{1-4}$-alkyl group,

$R^{12'}$ or $R^{13'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, hydrogen, methyl, ethyl, F, Cl, Br and $CF_3$,

$R^{14'}$ or $R^{15'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, methyl, ethyl, F, Cl, Br and $CF_3$, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also, preferably the pharmaceutical formulation according to the present invention comprises one or more substituted pyrazoline compounds of general formula I' given above, wherein in general structure II $R^{12'}$ and $R^{13'}$ independently of each other represent hydrogen, a linear or branched $C_{1-6}$-alkyl group, a halogen atom, or $CF_3$, preferably $R^{12'}$ and $R^{13'}$ independently of each other represent hydrogen, methyl, ethyl, F, Cl, Br and $CF_3$.

Also, preferably the pharmaceutical formulation according to the present invention comprises one or more substituted pyrazoline compounds of general formula I' given above, wherein in general structure II $R^{14'}$ and $R^{15'}$ independently of each other represent a linear or branched $C_{1-6}$-alkyl group, a halogen atom, or $CF_3$, preferably $R^{14'}$ and $R^{15'}$ independently of each other represent methyl, ethyl, F, Cl, Br and $CF_3$.

Also, preferably the pharmaceutical formulation according to the present invention comprises one or more substituted pyrazoline compounds of general formula I' given above, wherein in general structure II $R^{13'}$ represents Cl and $R^{12'}$ represents hydrogen.

Also, preferably the pharmaceutical formulation according to the present invention comprises one or more substituted pyrazoline compounds of general formula I' given above, wherein in general structure II $R^{14'}$ and $R^{15'}$ each represent Cl.

Also, preferably the pharmaceutical formulation according to the present invention comprises one or more substituted pyrazoline compounds of general formula I' given above, wherein in general structure II $R^{1'}$ represents hydrogen, methyl or ethyl, preferably hydrogen.

In another preferred an aspect of the invention relates to a pharmaceutical formulation comprising

(A) at least one substituted pyrazoline compound selected from the group consisting of
5-(4-chloro-phenyl)-1-(2,4-dichlorophenyl)-4,5-dihydro-1H-pyrazol-3-carboxylic acid,
(rac)-5-(4-chloro-phenyl)-1-(2,4-dichlorophenyl)-4,5-dihydro-1H-pyrazol-3-carboxylic acid,
(S)-5-(4-chloro-phenyl)-1-(2,4-dichlorophenyl)-4,5-dihydro-1H-pyrazol-3-carboxylic acid,
(R)-5-(4-chloro-phenyl)-1-(2,4-dichlorophenyl)-4,5-dihydro-1H-pyrazol-3-carboxylic acid,
optionally in form of a corresponding salt thereof, or a corresponding solvate thereof,
and

(B) nanoparticles,

wherein component (A) is associated with component (B).

Also, more preferably the pharmaceutical formulation according to the present invention may as the pyrazoline compound of general formula I' comprise one of the following compounds

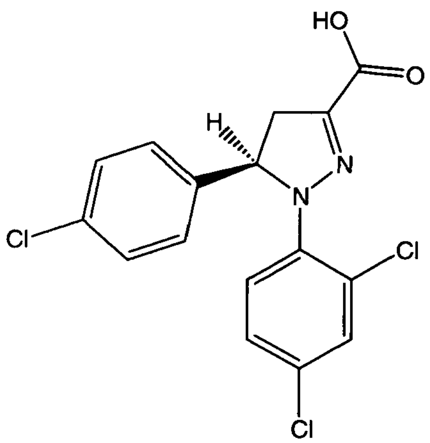

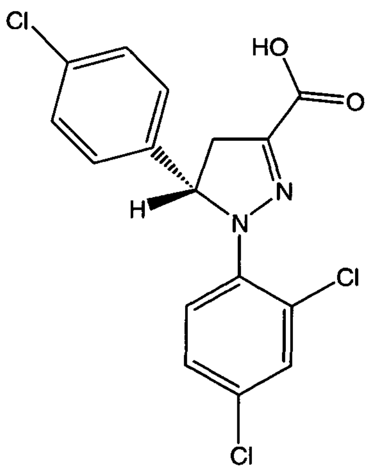

in each case optionally in the form of a corresponding N-oxide, a corresponding salt or a corresponding solvate thereof.

Here, in connection with alkyl and cycloalkyl - unless expressly defined otherwise - the term substituted in the context of this invention is understood as meaning replacement of at least one hydrogen radical by F, Cl, Br, I, $NH_2$, SH or OH, "polysubstituted" radicals being understood as meaning that the replacement takes effect both on different and on the same atoms several times with the same or different substituents, for example three times on the same C atom, as in the case of $CF_3$, or at different places, as in the case of -CH(OH)-CH=CH-$CHCl_2$. Particularly preferred substituents here are F, Cl and OH. In respect of cycloalkyl, the hydrogen radical can also be replaced by $OC_{1-3}$-alkyl or $C_{1-3}$-alkyl (in each case mono- or polysubstituted or unsubstituted), in particular methyl, ethyl, n-propyl, i-propyl, $CF_3$, methoxy or ethoxy.

The term $(CH_2)_{3-6}$ is to be understood as meaning $-CH_2-CH_2-CH_2-$, $-CH_2-CH_2-CH_2-CH_2-$, $-CH_2-CH_2-CH_2-CH_2-CH_2-$ and $-CH_2-CH_2-CH_2-CH_2-CH_2-CH_2-$, $(CH_2)_{1-4}$ is to be understood as meaning $-CH_2-$, $-CH_2-CH_2-$, $-CH_2-CH_2-CH_2-$ and $-CH_2-CH_2-CH_2-CH_2-$, $(CH_2)_{4-5}$ is to be understood as meaning $-CH_2-CH_2-CH_2-CH_2-$ and $-CH_2-CH_2-CH_2-CH_2-CH_2-$, etc.

An aryl radical is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

A heteroaryl radical is understood as meaning heterocyclic ring systems which have at least one unsaturated ring and can contain one or more heteroatoms from the group consisting of nitrogen, oxygen and/or sulfur and can also be mono- or polysubstituted. Examples which may be mentioned from the group of heteroaryls are furan, benzofuran, thiophene, benzothiophene, pyrrole, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

Here, in connection with aryl and heteroaryl, substituted is understood as meaning substitution of the aryl or heteroaryl by R, OR, a halogen, preferably F and/or Cl, a $CF_3$, a CN, an $NO_2$, an NRR, a $C_{1-6}$-alkyl (saturated), a $C_{1-6}$-alkoxy, a $C_{3-8}$-cycloalkoxy, a $C_{3-8}$-cycloalkyl or a $C_{2-6}$-alkylene.

The inventively used substituted pyrazoline compounds may, for example, be obtained by the following process, according to which which at least one benzaldehyde compound of general formula II

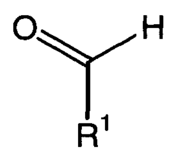

(II)

wherein $R^1$ has the meaning given above, is reacted with a pyruvate compound of general formula (III)

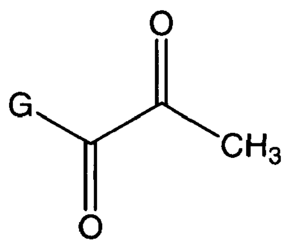

(III),

wherein G represents an OR group with R being a branched or unbranched $C_{1-6}$ alkyl radical, preferably an ethyl radical,

or G represents an O K group with K being a cation, preferably a monovalent cation, more preferably an alkali metal cation, even more preferably a sodium cation, to yield a compound of general formula (IV)

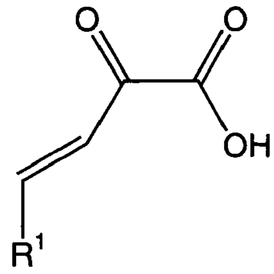

(IV)

wherein $R^1$ has the meaning given above, which is optionally isolated and/or optionally purified, and which is reacted with an optionally substituted phenyl hydrazine of general formula (V)

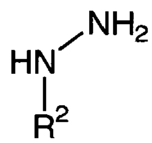

(V)

or a corresponding salt thereof, wherein $R^2$ has the meaning given above, under an inert atmosphere, to yield a compound of general formula (VI)

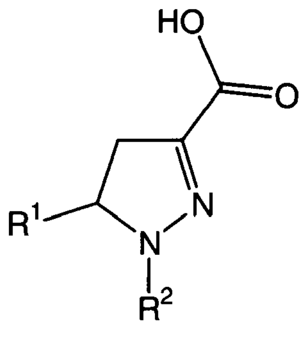

(VI)

wherein $R^1$ and $R^2$ have the meaning as given above, which is optionally isolated and/or optionally purified, and optionally transferred under inert atmosphere to a compound of general formula (VII)

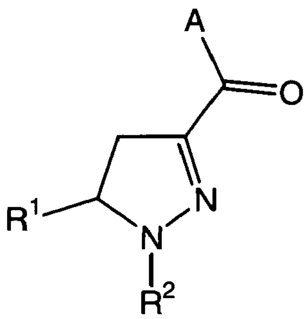

(VII)

wherein the substituents $R^1$ and $R^2$ have the meaning given above and A represents a leaving group, via the reaction with an activating agent, said compound being optionally isolated and/or optionally purified, and at least one compound of general formula (VI) is reacted with a compound of general formula $R^3H$, wherein $R^3$ represents an $-NR^4R^5$-moiety, wherein $R^4$ and $R^5$ have the meaning given above, to yield a substituted pyrazoline compound of general formula I, wherein $R^3$ represents an $-NR^4R^5$-moiety,
and/or at least one compound of general formula (VII) is reacted with a compound of the general formula $R^3H$, in which $R^3$ has the meaning given above to yield a compound of general formula (I) given above, which is optionally isolated and/or optionally purified.

The process is also illustrated in scheme I given below:

Scheme I:

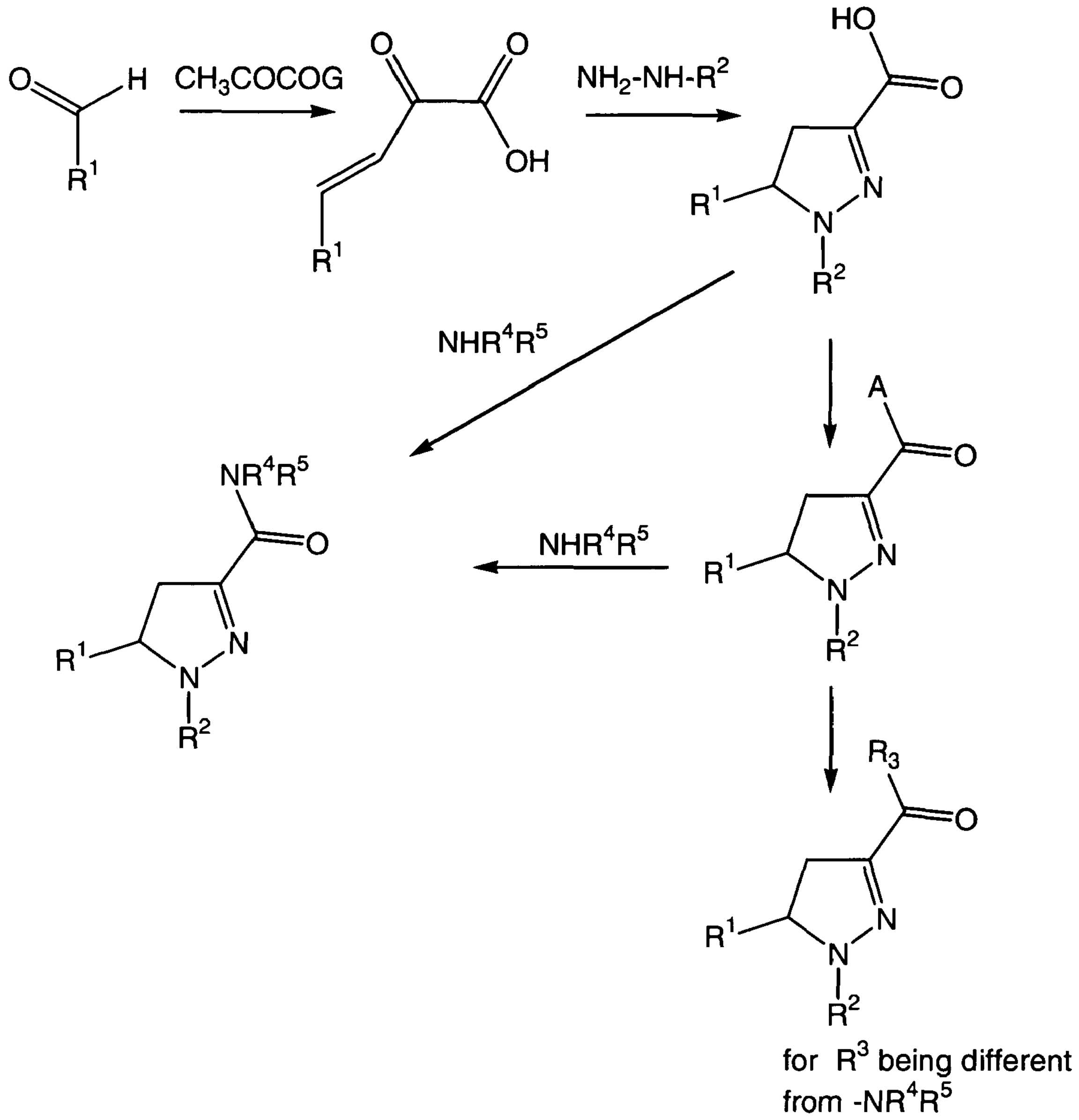


The reaction of the benzaldehyde compound of general formula II with a pyruvate compound of general formula III is preferably carried out in the presence of at least one base, more preferably in the presence of an alkali metal hydroxide such as sodium hydroxide or potassium hydroxide or an alkali metal methoxide such as sodium methoxide, as described, for example, in Synthetic communications, 26(11), 2229-33, (1996). The respective description is hereby incorporated by reference and forms part of the disclosure. Preferably sodium pyruvate may be used as the pyruvate compound. Preferably said reaction is carried out in a protic reaction medium such as a $C_{1-4}$ alkyl alcohol or mixtures of these. Mixtures of such alcohols with water, e.g. ethanol/water may also be used.

Reaction temperature as well as the duration of the reaction may vary over a broad range. Preferred reaction temperatures range from -10 °C to the boiling point of the reaction medium. Suitable reaction times may vary for example from several minutes to several hours.

Also preferred the reaction of the benzaldehyde compound of general formula II with a pyruvate compound of general formula III is carried out under acid catalysed conditions, more preferably by refluxing the mixture in dichloromethane in the presence of copper(II)trifluoromethanesulfonate as described, for example, in Synlett, (1), 147-149,

2001. The respective description is hereby incorporated by reference and forms part of the disclosure.

The reaction of the compound of general formula (IV) with an optionally substituted phenyl hydrazin of general formula (V) is preferably carried out in a suitable reaction medium such as $C_{1-4}$-alcohols or ethers such as dioxane or tetrahydrofurane or mixtures of at least two of these afore mentioned compounds. Also preferably, said reaction may be carried out in the presence of an acid, whereby the acid may be organic such as acetic acid and/or inorganic such as hydrochloric acid. Furthermore, the reaction may also be carried out in the presence of a base such as piperidine, piperazine, sodium hydroxide, potassium hydroxide, sodium methoxide or sodium ethoxide, or a mixture of at least two of these bases may also be used.

Reaction temperature as well as the duration of the reaction may vary over a broad range. Suitable reaction temperatures range from room temperature, i.e. approximately 25 °C to the boiling point of the reaction medium. Suitable reaction times may vary for example from several minutes to several hours.

The carboxylic group of the compound of general formula (VI) may be activated for further reactions by the introduction of a suitable leaving group according to conventional methods well known to those skilled in the art. Preferably the compounds of general formula (VI) are transferred into an acid chloride, an acid anhydride, a mixed anhydride, a $C_{1-4}$ alkyl ester, an activated ester such as p-nitrophenylester. Other well known methods for the activation of acids include the activation with N,N-dicyclohexylcarbodiimide or benzotriazol-N-oxotris(dimethylamino) phosphonium hexafluorophosphate (BOP)).

If said activated compound of general formula (VII) is an acid chloride, it is preferably prepared by reaction of the corresponding acid of general formula (VI) with thionyl chloride or oxalyl chloride, whereby said chlorinating agent is also used as the solvent. Also preferably an additional solvent may be used. Suitable solvents include hydrocarbons such as benzene, toluene or xylene, halogenated hydrocarbons such as dichloromethane, chloroform or carbon tetrachloride, ethers such as diethyl ether, dioxane, tetrahydrofurane or dimethoxyethane. Mixtures of two or more solvents from one class or two or more solvents from different classes may also be used. Preferred reaction temperature range from 0º C to the boiling point of the solvent and reaction times from several minutes to several hours.

If said activated compound of general formula (VII) is a mixed anhydride, said anhydride may preferably be prepared, for example, by reaction of the corresponding acid of general formula (VI) with ethyl chloroformiate in the presence of a base such as triethylamine or pyridine, in a suitable solvent.

The reaction of general formula (VII) with a compound of general formula $HR^3$ to yield compounds of general general I, wherein $R^3$ represents an $-NR^4R^5$ moiety is preferably carried out in presence of a base such as triethylamine in a reaction medium such as methylenchloride. The temperature is preferably in the range from 0ºC to the boiling point of the reaction medium. The reaction time may vary over a broad range, e.g. from several hours to several days.

The reaction of general formula (VII) with a compound of general formula $HR^3$ to yield compounds of general formula I, wherein $R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system may be carried out according to conventional methods well known to those skilled in the art, e.g. from Pascual, A., J. Prakt Chem., 1999, 341 (7), 695-700; Lin, S. et al., Heterocycles, 2001, 55(2), 265-277; Rao, P. et al., J. Org. Chem., 2000, 65(22), 7323-7344, Pearson D.E and Buehler, C.A., Synthesis, 1972, 533-542 and references cited therein. The respective descriptions are hereby incorporated by reference and form part of the present disclosure.

Preferably said reaction is carried out in the presence of a Lewis acid, which is preferably selected from the group consisting of $FeCl_3$, $ZnCl_2$ and $AlCl_3$, in a suitable reaction medium such as toluene, benzene, tetrahydrofurane or similar. The temperature is preferably in teh range from 0ºC to the boiling point of the reaction medium, more preferably from 15 to 25 °C. The reaction time may vary over a broad range, e.g. from several minutes to several hours.

The afore mentioned reactions involving the synthesis of the 4,5-dihydro-pyrazole ring or the reaction of a compound comprising said ring are carried out under an inert atmosphere, preferably nitrogen or argon, to avoid oxidation of the ring-system.

During the processes described above the protection of sensitive groups or of reagents may be necessary and/or desirable. The introduction of conventional protective groups as well as their removal may be performed by methods well-known to those skilled in the art.

If the substituted pyrazoline compounds of general formula (I) themselves are obtained in form of a mixture of stereoisomers, particularly enantiomers or diastereomers, said mixtures may be separated by standard procedures known to those skilled in the art, e.g. chromatographic methods or fractunalized crystallization with chiral reagents. It is also possible to obtain pure stereoisomers via stereoselective synthesis.

Salts of the inventively used active compounds may be obtained by a process, wherein at least one of the compounds having at least one basic group is reacted with at least one inorganic and/or organic acid, preferably in the presence of a suitable reaction medium. Suitable reaction media include, for example, any of the ones given above. Suitable inorganic acids include hydrochloric acid, hydrobromic acid, phosphoric acid, sulfuric acid, nitric acid, suitable

organic acids are e.g. citric acid, maleic acid, fumaric acid, tartaric acid, or derivatives thereof, p-toluenesulfonic acid, methanesulfonic acid or camphersulfonic acid.

Salts of the inventively used active compounds may also be prepared by a method, wherein at least one of the compounds having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. $[NH_nR_{4-n}]^+$, wherein n is 0, 1, 2, 3 or 4 and R represents a branched or unbranched $C_{1-4}$-alkyl-radical. Suitable reaction media are, for example, any of the ones given above.

Solvates, preferably hydrates, of the inventively used substituted pyrazoline compounds of general formula (I), of corresponding stereoisomers, of corresponding N-oxides or of corresponding salts thereof may also be obtained by standard procedures known to those skilled in the art.

Substituted pyrazoline compounds of general formula I, which comprise nitrogen-atom containing saturated, unsaturated or aromatic rings may also be obtained in the form of their N-oxides by methods well known to those skilled in the art and used in the active substance combination of the present invention.

The purification and isolation of the inventive substituted pyrazoline compounds of general formula (I), of a corresponding stereoisomer, or salt, or N-oxide, or solvate or any intermediate thereof may, if required, be carried out by conventional methods known to those skilled in the art, e.g. chromatographic methods or recrystallization.

The substituted pyrazoline compounds of general formula (I) given below, their stereoisomers, corresponding N-oxides, corresponding salts thereof and corresponding solvates are toxicologically acceptable and are therefore suitable as pharmaceutical active substances for the preparation of medicaments.

It has been found that the substituted pyrazoline compounds of general formula I given below, stereoisomers thereof, N-oxides thereof, corresponding salts and corresponding solvates have a high affinity to cannabinoid receptors, particularly cannabinoid 1 ($CB_1$)-receptors, i.e. they are selective ligands for the ($CB_1$)-receptor and act as modulators, e.g. antagonists, inverse agonists or agonists, on these receptors. In particular, these pyrazoline compounds show little or no development of tolerance during treatment, particularly with respect to food intake, i.e. if the treatment is interrupted for a given period of time and then continued afterwards, the inventively used pyrazoline compounds will again show the desired effect. After ending the treatment with the pyrazoline compounds, the positive influence on the body weight is found to continue.

Furthermore, these pyrazoline compounds show relatively weak Herg channel affinity, thus a low risk of prolongation of the QT-interval is to be expected for these compounds.

In summary, the inventively used pyrazoline compounds are distinguished by a broad spectrum of beneficial effects, while at the same time showing relatively little undesired effects, i.e. effects which do not positively contribute to or even interfere with the well being of the patient.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic-especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

In connection with this invention "neutral form" refers to the non-ionic form but also to (at its isoelectric point) neutrally charged forms (that means containing an equal amount of positive and negative charges) especially the Zwitter-

Ion.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by $^{13}C$- or $^{14}C$-enriched carbon or $^{15}N$-enriched nitrogen are within the scope of this invention.

A preferred aspect of the present invention is a pharmaceutical formulation comprising the compound, its derivative or its analogue, in neutral form or as a salt, especially a physiologically acceptable salt.

A preferred aspect of the present invention is a pharmaceutical formulation comprising a substituted pyrazoline compound as defined herein, covalently bound to nanoparticles.

A preferred aspect of the present invention is a pharmaceutical formulation wherein the component (A) is covalently bound to component (B).

Another preferred aspect of the present invention is a pharmaceutical formulation wherein component (A) is bound to component (B) via van der Waals interaction, ionic interaction or hydrogen bonding.

Another preferred aspect of the present invention is a pharmaceutical formulation wherein component (A) forms a suspension or emulsion with component (B).

Another preferred aspect of the present invention is a pharmaceutical formulation wherein component (A) is enclosed in component (B).

Another preferred aspect of the present invention is a pharmaceutical formulation wherein component (A) is complexed, absorbed or adsorbed by component (B).

Another preferred aspect of the present invention is a pharmaceutical formulation wherein said formulation is administered orally or parenterally.

In a preferred aspect of the present invention the pharmaceutical formulation is an emulsion

A preferred aspect of the present invention is a pharmaceutical formulation component (A) is entrapped or encapsulated in component (B).

In a preferred embodiment of the present invention said nanoparticles have a diameter of below 1,000nm, preferably of from 1 to 1,000nm.

In another preferred embodiment of the invention said nanoparticles cross the blood-bain barrier.

In another aspect of the present invention, said nanoparticles comprise one or more materials selected from the group consisting of polyacrylates, polymethacrylates, poly-cyanoacrylates, polyacrylamides, polylactates, polyglycolates, polyanhydrates, polyorthoesters, gelatin, polysaccharides, albumin, polystyrenes,polyvinyls, polyacrooein, polyglutaraldehydes and derivatives, copolymers and mixtures thereof.

Preferrably, a component of the nanoparticles used in the present invention is/are a stabilizer (s)/surfactants. In a preferred use, optionally at least one stabilizer and/or surfactant is used.

In another preferred embodiment of the present invention, said stabilizers/surfactants is/are a substance selected from the group consisting of polysorbates, dextrans, carboxylic acid esters of multifunctional alcohols, polyoxamers, polyoxamines, alkoxylated ethers, alkoxylated esters, alkoxylated mono-, di-and triglycerides, alkoxylated phenols and diphenols, substances of the Genapol R and Bauki R series, metal salts of carboxylic acids, metal salts of alcohol sulfates, and metal salts of sulfosuccinates and mixtures of two or more of said substances, more preferably wherein said stabilizer/surfactant comprises a substance selected from the group consisting of polysorbate 20, polysorbate 40, polysorbate 60, polysorbate 80, polysorbate 81, polysorbate 85, dextran 12.000, dextran 70,000, fatty acid esters of glycerol and sorbitol as glycerol monostearate, sorbitan monostearate and sorbitan monooleate, polyoxamer 188 (Pluronic R F68), ethoxylated ethers, ethoxylated esters, ethoxylated triglycerides, ethoxylated phenols and diphenols, metal salts of fatty acids and of fatty alcohol sulfates, more preferably the sodium salts of fatty acids and of fatty alcohol sulfates, even more preferably sodium stearate and sodium lauryl sulfate, and mixtures of two or more of said substances, even more preferably polysorbate 80, polysorbate 85, dextran 12,000 or dextran 70,000 and mixtures thereof and mixtures of the stabilizers/surfactants with otherstabilizers/surfactants.

In another preferred embodiment of the present invention, the material (s) of the stabilizer/ surfactant is/are selected selected from the group consisting of stabilizers/surfactants which allow a passage of said nanoparticles including said physiologically effective substance (s) across the blood brain barrier in said mammal and stabilizers/surfactants which allow a release of said physiologically effective substance (s) from said nanoparticles and a passage of said substance (s) across the blood brain barrier separate from said nanoparticles. It is furthermore preferred that said stabilizer/surfactant comprises a substance selected from the group consisting of polysorbates, dextrans, carboxylic acid esters of multifunctional alcohols, polyoxamers,polyoxamines, alkoxylated ethers, alkoxylated esters, alkoxylated mono-, di-and triglycerides, alkoxylated phenols and diphenols, substances of the GenapolR and Bauki R series, metal salts of carboxylic acids, metal salts of alcohol sulfates, and metal salts ofsulfosuccinates and mixtures of two or more of said substances.

In connection with this invention "pharmaceutical formulation" means a pharmaceutical formulation or a pharmaceutical composition in which the parts and ingredients including the active compound/s (ingredient/s) or — (preferably

but not necessarily) optionally - auxiliary material/s and/or additive/s are designed to be used therapeutically in the treatment of a patient like e.g a tablet, an injectable fluid, a cream or a patch.

In connection with this invention the term "compound" also means the same as "active principle" or "active ingredient".

The pharmaceutical formulations or pharmaceutical compositions according to the invention contain the active ingredient as well as — (preferably but not necessarily) optionally - at least one auxiliary material and/or additive. The auxiliary material and/or additive can be selected from carrier, excipient, support materials, glidants, fillers, solvents, diluents, colorants, taste conditioners like sugars, antioxidants and/or binders. The selection of these auxiliary materials and/or additives and of the amounts to be used depends upon how the pharmaceutical composition is to be applied.

In connection with this invention an oral formulation refers to a pharmaceutical formulation which is designed to be taken or consumed orally by a patient and/or if used for a therapeutic use will have to enter the body through the mouth and oesophagus.

"Topical formulations", as referred to in this invention, can be in any form suitable for application to the body surface, and may comprise, for example, a fluid, a gel, an ointment, a foam, a balm, a lotion, a cream, a sleeve, a patch or any type of recipient which can be adhered to the skin, store and/or release active ingredient. "Body surface" in this invention refers to any type of epithelium, e.g. nasal epithelium. Therefore, formulations such as a spray or an inhaler are included in the category of topical administration.

Patch formulations according to the present invention refer to adhesive devices attached to any type of epithelium surface, storing and releasing the active ingredient either in an immediately release or controlled relase-dependent manner.

In the context of this invention "controlled release" formulation which is to be used synonymously with "slow release" formulation, "sustained-release" formulation or "timed release" formulation means any formulation with a release profile from which measured according to a standard measurement (e.g. using the paddle method according to the Pharmacopeia) (e.g. in 0.1% NaCl solution) within 30 minutes less than 50 %, more preferably less than 40 %, or even more preferably less than 30 % of the active compound is released.

In a preferred embodiment of the invention the pharmaceutical formulation according to the invention is a controlled-release formulation.

In a preferred embodiment of the invention the pharmaceutical formulation according to the invention is a sustained-release formulation.

In the context of this invention "immediately release" formulation means any pharmaceutical formulation with a release profile from which measured according to a standard measurement (e.g. using the paddle method according to the Pharmacopeia) (e.g. in 0.1% NaCl solution) within 30 minutes more than 50 %, more preferably 60 %, or even more preferably 70 % of the active compound is released.

In a preferred embodiment of the invention the pharmaceutical formulation according to the invention is an immediate release formulation.

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

In another preferred embodiment of the invention the parenteral administration of said medicament containing the formulation includes intramuscular injection, intraveneous injection, subcutaneous injection, collagen implants, aerosols, suppositories, implantable osmotic pumps, or a patches through epithelial surface, e.g. skin.

In another preferred embodiment of the invention a patch contains the active ingredient assiciated nanoparticles, either in solid form, in form of a solution, a fluid, a gel, an ointment, an emulsion, a foam, a balm, a lotion, or a cream Pharmacological Methods

**I. In-vitro determination of affinity to CB1/CB2-Receptors**

The in-vitro determination of the affinity of the inventive substituted pyrazoline compounds to $CB_1$/$CB_2$-Rezeptors is carried out as described in the publication of Ruth A. Ross, Heather C. Brockie et al., "Agonist-inverse agonist characterization at CB1 and CB2 cannabinoid receptors of L-759633, L759656 and AM630", British Journal of Pharmacology, 126, 665-672, (1999), whereby the transfected human $CB_1$ and $CB_2$ receptors of Receptor Biology, Inc. are used. The radioligand used for both receptors is [$^3$H]-CP55940. The respective parts of the description is hereby incorporated by reference and forms part of the present disclosure.

## II. In-vivo bioassay system for determination of cannabinoid activity

### Mouse tetrad model

Substances with affinity for cannabinoid receptors are known to produce a wide range of pharmacological effects. It is also known that intravenous administration of a substance with affinity for cannabinoid receptors in mice produces analgesia, hypothermia, sedation and catalepsy. Individually, none of these effects can be considered as proof that a tested substance has affinity for cannabinoid-receptors, since all of these effects are common for various classes of centrally active agents. However, substances, which show all of these effects, i.e. substances that are active in this so-called tetrad model are considered to have affinity for the cannabinoid receptors. It has further been shown that cannabinoid receptor antagonists are higly effective in blocking the effects of a cannabinoid agonist in the mouse tetrad model.

The tetrad model is described, for example, in the publication of A. C. Howlett et al, International Union of Pharmacology XXVII. Classification of Cannabinoid Receptors, Pharmacol Rev 54, 161-202 , 2002 and David R. Compton et al., "In-vivo Characterization of a Specific Cannabinoid Receptor Antagonist (SR141716A) :Inhibition of Tetrahydrocannbinol- induced Responses and Apparent Agonist Activity", J. Pharmacol. Exp. Ther. 277 , 2, 586-594, 1996. The corresponding parts of the description are hereby incorporated by reference.

### Material and Methods

Male NMRI mice with a weight of 20-30 g (Harlan, Barcelona, Spain) are used in all of the following experiments.

Before testing in the behavioral procedures given below, mice are acclimatized to the experimental setting. Pre-Treatment control values are determined for analgesia hot plate latency (in seconds), rectal temperature, sedation and catalepsy.

In order to determine the agonistic activty of the substance to be tested, the mice are injected intravenously with the substance to be tested or the vehicle alone. 15 minutes after injection, latency in hot plate analgesia is measured.

Rectal temperature, sedation and catalepsy are measured 20 minutes after injection.

In order to determine the antagonistic activity the identical procedure is used as for the determination of the agonistic effects, but with the difference that the substance to be evaluated for its antagonistic activity is injectected 5 minutes before the intravenous injection of 1.25 mg/kg Win-55,212 a known cannabinoid-receptor agonist.

### Hot plate analgesia

The hot plate analgesia is determined according to the method described in Woolfe D. et al. "The evaluation of analgesic action of pethidine hydrochloride (Demerol)", J. Pharmacol. Exp. Ther. 80, 300-307,1944. The respective description is hereby incorporated by reference and forms part of the present disclosure.

The mice are placed on a hot plate (Harvard Analgesimeter) at 55 $\pm$ 0.5 °C until they show a painful sensation by licking their paws or jumping and the time for these sensations to occur is recorded. This reading is considered the basal value (B). The maximum time limit the mice are allowed to remain on the hot plate in absence of any painful response is 40 seconds in order to prevent skin damage. This period is called the cut-off time (PC).

Fifteen minuts after the administration of the substance to be tested, the mice are again placed on the hot plate and the afore described procedure is repeated. This period is called the post-treatment reading (PT).

The degree of analgesia is calculated from the formula :

$$\%\ \text{MPE of Analgesia} = (PT - B) / (PC - B) \times 100$$

MPE = Maximum possible effect.

### Determination of sedation and ataxia

Sedation and ataxia is determined according to the method described in Desmet L. K. C. et al. "Anticonvulsive properties of Cinarizine and Flunarizine in Rats and Mice", Arzneim. -Forsch. (Frug Res) 25, 9, 1975. The respective description is hereby incorporated by reference and forms part of the present disclosure.

The chosen scoring system is

0: no ataxia;

1: doubful;
2: obvious calmness and quiet;
3 pronounced ataxia;
prior to as well as after treatment.
The percentage of sedation is determined according to the formula:

$$\% \text{ of sedation} = \text{arithmetic mean} / 3 \times 100$$

**Hypothermia:**

Hypothermia is determined according to the method described in David R. Compton et al. "In-vivo Characterization of a Specific Cannabinoid Receptor Antagonist (SR141716A) Inhibition of Tetrahydrocannbinol- induced Responses and Apparent Agonist Activity", J. Pharmacol Exp Ther. 277 , 2, 586-594, 1996.
The respective description is hereby incorporated by reference and forms part of the present disclosure.
The base-line rectal temperatures are determined with a thermometer (Yello Springs Instruments Co., Panlabs) and a thermistor probe inserted to 25mm before the administration of the substance to be tested. Rectal temperature is again measured 20 minutes after the administration of the substances to be tested. The temperature difference is calculated for each animal, whereby differences of ≥ -2 ºC are considered to represent activity.

**Catalepsy:**

Catalepsy is determined according to the method described in Alpermann H. G. et al. "Pharmacological effets of Hoe 249: A new potential antidepressant", Drugs Dev. Res. 25, 267-282. 1992. The respective description is hereby incorporated by reference and forms part of the present disclosure.
The cataleptic effect of the substance to be tested is evaluated according to the duration of catalepsy, whereby the animals are placed head downwards with their kinlegs upon the top of the wooden block.

The chosen scoring system is:

Catalepsy for:
more than 60 seconds = 6; 50 -60 seconds = 5, 40-50 seconds = 4, 30-40 seconds = 3, 20-30 seconds = 2, 5-10 seconds = 1, and less than 5 seconds =0.
The percentage of catalepsy is determined according ot the following formula:

$$\% \text{ Catalepsy} = \text{arithmetic mean} / 6 \times 100$$

**III. In vivo testing for antiobesic activity**

The in-vivo testing for antiobesic activity of the inventive pyrazoline compounds is carried out as described in the publication of G. Colombo et al., "Appetite Suppression and Weight Loss after the Cannabinoid Antagonist SR 141716"; Life Sciences, 63 (8), 113-117, (1998). The respective part of the description is hereby incorporated by reference and forms part of the present disclosure.

**IV. In vivo testing for antidepressant activity**

The in-vivo testing for antidepressant activity of the inventive pyrazoline compounds in the water despair test is carried out as described in the publication of E.T. Tzavara et al., "The CB1 receptor antagonist SR141716A selectively increases monoaminergic neurotransmission in the medial prefrontal cortex: implications for therapeutic actions"; Br. J. Pharmacol. 2003, 138(4):544:53. The respective part of the description is hereby incorporated by reference and forms part of the present disclosure.
The present invention is illustrated below with the aid of examples. These illustrations are given solely by way of example and do not limit the general spirit of the present invention.
The compound according to example 0 is an inhibitor of high blood levels of triglicerides. This effect has been probed in obese mice fed with high fat diet. In the following paragraphs it is described the method and the results obtained

in this study.

**V. In-vivo testing for regulation of triglycerides in blood plasma**

The study was done using six weeks old male mice B6 Lep ob/ob, obtained from Charles River (France). Mice were divided in 3 groups : I (control), II (vehicle), III (example 0).

Group I:

The animals of the group I received the standard diet (D-12450B, Research Diets, NJ, USA).

Group II:

The animals of the groups II and III were fed with a High Fat Diet (D-12492, Research Diets, NJ, USA), in both cases for 7 weeks (References 1 and 2).

Group III:

The animals of the groups III were fed with a High Fat Diet (D-12492, Research Diets, NJ, USA), in both cases for 7 weeks (References 1 and 2).
At the end of the feeding period of 7 weeks, it was started the treatment period (14 days): Group II mice received the vehicle (10 ml/kg/day, po, of the aqueous solution of acacia gum, 5% WN). Group III was administered with 30 mg/kg/day, po, of the inventive compound 5-(4-chlorophenyl)-1-(2,4-dichlorophenyl)-4,5-dihydro-pyrazole-3-carboxylic acid according to Example 0. Group I didn't received any treatment. The three groups of mice had the same diet than in the previous period.
At the end of the 14 days period of treatment, the blood levels of triglicerides of the animals were determined.
The analysis of the whole blood samples was done using test strips "Lipid panel" and the photometric Analyzer Cardio-Check Test System, from PA Instruments Polymer Technology Systems Indianapolis, IN-46268, USA (Distributed in Spain by Novalab Iberica S.A.L, Madrid, Spain).

**VI. In-vivo testing for regulation of triglycerides in blood plasma**

In a second set of experiments carried out similar to the tests shown above the TG (triglyceride) levels of diet-induced obese mice in blood were determined.
Mice receiving a high fat diet were - after a feeding period of 6 days - either treated p.o. with vehicle (0,5 % HPMC) or with the compound according to example 0 (30 mg/kg/day p.o.).
TG levels in blood were determined on day 28 after beginning of the treatment.

**Examples:**

**Example 1:**

N-piperidinyl-5-(4-chlorophenyl)-1-(2,4-dichlorophenyl)-4,5-dihydro-1H-pyrazole-3-carboxamide

a) 4-(4-chlorophenyl)-2-oxo-3-butenoic acid

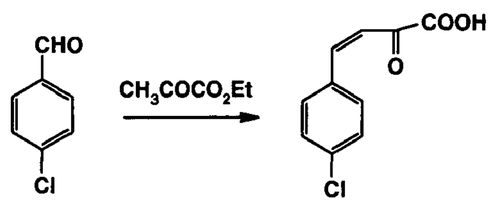


In a three neck flask p-chlorobenzaldehyde (13,3 g, 95 mmoles) and ethyl pyruvate (10 g, 86 mmoles) were dissolved in 150 ml of absolute ethanol.The solution was ice-cooled to 0°C and an aqueous solution of NaOH (3.8 g in 45 mL water) was added dropwise keeping the temperature below or equal to 10°C, whereby a yellow-orange colored precipitate was formed. The reaction mixture was stirred for 1 hour at 0°C and an additional 1.5 hours at room

temperature (approximately 25 °C). Afterwards the reaction mixture was cooled down to approximately 5°C and the insoluble sodium salt of 4-(4-chlorophenyl)-2-oxo-3-butenoic acid was isolated by filtration.

The filtrate was left in the refrigerator overnight, whereby more precipitate is formed, which was filtered off, combined with the first fraction of the salt and washed with diethyl ether. The sodium salt of 4-(4-chlorophenyl)-2-oxo-3-butenoic acid was then treated with a solution of 2N HCl, stirred for some minutes and solid 4-(4-chlorophenyl)-2-oxo-3-butenoic acid was separated via filtration and dried to give 12.7 g of the desired product (70% of theoretical yield).

IR (KBr, $cm^{-1}$) : 3500-2500, 1719,3, 1686,5, 1603,4, 1587,8, 1081,9.

$^1$H NMR($CDCl_3$, δ) : 7,4 (d, J=8,4Hz, 2H), 7,5 (d, J=16,1Hz, 1 H), 7,6 (d, J=8,4Hz, 2H), 8,1 (d, J=16,1 Hz, 1 H).

b) 5-(4-chlorophenyl)-1-(2,4-dichlorophenyl)-4,5-dihydro-pyrazole-3-carboxylic acid

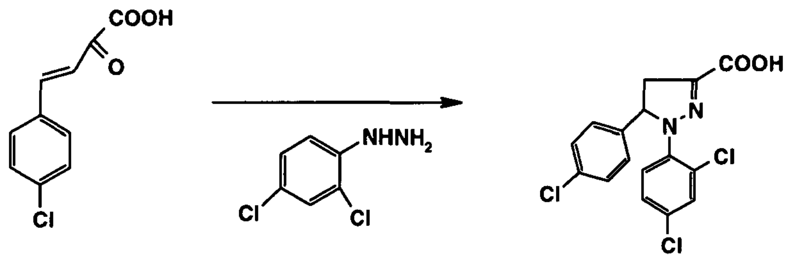

4-(4-chlorophenyl)-2-oxo-3-butenoic acid obtained according to step a) (12.6 g, 60 mmoles), 2,4-dichlorophenylhydrazine hydrochloride (12.8 g, 60 mmoles) and glacial acetic acid (200 mL) were mixed under a nitrogen atmosphere and heated to reflux for 4 hours, cooled down to room temperature (approximately 25 °C) and given into ice-water, whereby a sticky mass was obtained, which was extracted with methylene chloride. The combined methylene chloride fractions were washed with water, dried with sodium sulfate, filtered and evaporated to dryness to give a pale yellow solid (12.7 g, 57% of theoretical yield).

IR (KBr, $cm^{-1}$) : 3200-2200, 1668,4, 1458, 1251,4, 1104,8.

$^1$H NMR ($CDCl_3$, δ) : 3,3 (dd, 1 H), 3,7 (dd, 1 H), 5,9 (dd, 1 H), 7,09-7,25 (m, 7H).

(c) 5-(4-chlorophenyl)-1-(2,4-dichlorophenyl)-4,5-dihydro-pyrazole-3-carboxylic acid chloride

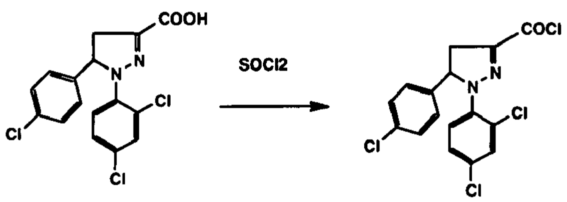


Under nitrogen atmosphere 5-(4-chlorophenyl)-1-(2,4-dichlorophenyl)-4,5-dihydro-pyrazole-3-carboxylic acid (2.5 g, 6.8 mmols) obtained according to step (b) was dissolved in 4 mL of in thionyl chloride and heated to reflux for 2.5 hours. The excess thionyl chloride is removed from the reaction mixture under reduced pressure and the resulting crude residue (2.6 g) is used without any further purification.

IR (KBr, $cm^{-1}$) : 1732,3, 1700, 1533,3, 1478,1, 1212,9, 826,6.

d) N-piperidinyl-5-(4-chlorophenyl)-1-(2,4-dichlorophenyl)-4,5-dihydropyrazole-3-carboxamide [this compound may also be referred to as 5-(4-Chloro-phenyl)-1-(2,4-dichloro-phenyl)-4,5-dihydro-1H-pyrazole-3-carboxylic acid piperidin-1-ylamide or as 1-(2,4-dichlorophenyl)-5-(4-chlorophenyl)-4,5-dihydro-N-(piperidin-1-yl)-1H-pyrazole-3-carboxamide]

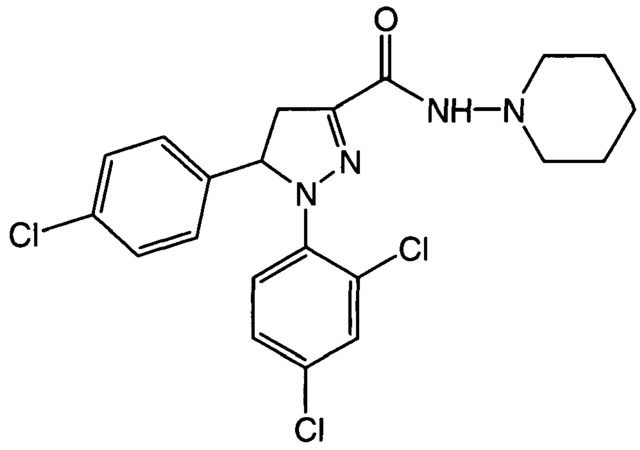

Under nitrogen atmosphere N-aminopiperidine (0.6 mL, 5.6 mmoles) and triethylamine (4 mL) were dissolved in methylene chloride (25 mL). The resulting mixture was ice-cooled down to 0°C and a solution of 5-(4-chlorophenyl)-1-(2,4-dichlorophenyl)-4,5-dihydro-pyrazole-3-carboxylic acid chloride obtained in step (c) in methylene chloride (15 mL) was added dropwise. The resulting reaction mixture was stirred at room temperature (approximately 25 °C) overnight. Afterwards the reaction mixture was washed with water, followed by a saturated aqueous solution of sodium bicarbonate, then again with water, dried over sodium sulfate, filtered and evaporated to dryness in a rotavapor. The resulting crude solid was crystallized from ethanol. The crystallized solid was removed via filtration and the mother liquors were concentrated to yield a second fraction of crystallized product. The two fractions were combined to give a total amount of 1.7 g (57% of theoretical yield) of N-piperidinyl-5-(4-chlorophenyl)-1-(2,4-dichlorophenyl)-4,5-dihydropyrazole-3-carboxamide having a melting point of 183-186°C.

IR (KBr, $cm^{-1}$) : 3222,9, 2934,9, 1647,4, 1474,7, 1268,3, 815,6.

$^{1}H$ NMR ($CDCl_3$, $\delta$) : 1,4 (m, 2H), 1,7 (m, 4H), 2,8 (m, 4H), 3,3 (dd, J=6,1 y 18,3Hz, 1 H), 3,7 (dd, J=12,5 and 18,3 Hz, 1 H), 5,7 (dd, J=6,1 and 12,5 Hz, 1 H), 7,0-7,2 (m, 6H), 7,4 (s, 1H).

The compounds according to the following examples 2-6 have been prepared analogously to the process described in Example 1.

**Example 2:**

5-(4-Chloro-phenyl)-1-(2,4-dichloro-phenyl)-4,5-dihydro-1H-pyrazole-3-carboxylic acid-[1,2,4]triazol-4-yl amide

Melting point: 134-138 ºC.

IR (KBr, $cm^{-1}$): 3448, 1686, 1477, 1243, 1091, 821.

$^{1}H$ NMR($CDCl_3$, $\delta$): 3,1 (dd, J=6,2 and 17,9Hz, 1H), 3,7 (dd, J=12,3 and 17,9Hz, 1 H), 5,9 (dd, J=6,2 and 12,3 Hz, 1 H), 7,2-7,5 (m, 7H), 8,7 (s, 2H), 12,0 (bs, 1H).

**Example 3:**

5-(4-Chloro-phenyl)-1-(2,4-dichloro-phenyl)-4,5-dihydro-1H-pyrazole-3-carboxylic acid-(4-methyl-piperazin-1-yl)-amide hydrochloride

Melting point: 150-155ºC.

IR (KBr, $cm^{-1}$) : 3433, 1685, 1477, 1296, 1246, 1088, 1014, 825.

$^{1}H$ NMR ($CDCl_3$, $\delta$): 2,7 (d, J=4,2Hz, 3H), 3,0-3,4 (m, 9H), 3,6 (dd, J=11,9 and 17,9 Hz, 1 H), 5,8 (dd, J=5,5 and 11,9 Hz, 1 H), 7,1 (d, J=8,4Hz, 2H), 7,25 (2d, J= 8,4 and 8,7 Hz, 3H), 7,4 (d, J=2,2Hz, 1 H), 7,5 (d, J=8,7Hz, 1 H), 9,8 (s, 1 H), 11,2 (bs).

**Example 4:**

5-(4-Chloro-phenyl)-1-(2,4-dichloro-phenyl)-4,5-dihydro-1H-pyrazole-3-carboxylic acid diethylamide

This compound was obtained in form of an oil.

IR (film, $cm^{-1}$) : 2974, 1621, 1471, 1274, 1092, 820.

$^1H$ NMR ($CDCl_3$, δ): 1,2 (m, 6H), 3,3-3,9 (m, 6H), 5,6 (dd, J=5,8 and 11,7 Hz, 1 H), 7-7,25 (m, 7H).

**Example 5:**

[5-(4-Chloro-phenyl)-1-(2,4-dichloro-phenyl)-4,5-dihydro-1H-pyrazol-3-yl]-piperidin-1-yl-methanone

Melting point: 105-110°C.

IR (KBr, $cm^{-1}$) : 2934, 1622, 1470, 1446, 1266, 1010, 817.

$^1H$ NMR ($CDCl_3$, δ): 1,7 (m, 6H), 3,4 (dd, J=5,7 and 17,9Hz, 1 H), 3,7 (m, 3H), 3,9 (m, 2H), 5,6 (dd, J=6,1 y 11,9 Hz, 1 H), 7-7,25 (m, 7H).

**Example 6:**

N-[5-(4-Chloro-phenyl)-1-(2,4-dichloro-phenyl)-4,5-dihydro-1H-pyrazole-3-carbonyl]-4-methyl-phenylsulfonamide

This compound was obtained in form of an amorph solid.

IR (KBr, $cm^{-1}$) : 1697, 1481, 1436, 1340, 1169, 1074, 853.

$^1H$ NMR ($CDCl_3$, δ): 2,4 (s, 3H), 3,2 (dd, J=6,6 and 18,3Hz, 1 H), 3,6 (dd, J=12,8 and 18,3Hz, 1 H), 5,8 (dd, J=6,6 and 12,8Hz, 1 H), 7 (d, J=8,2Hz, 2H), 7,2 (s, 1H), 7,3-7,4 (m, 6H), 8 (d, J=8,1 Hz, 2H), 9 (s, 1H).

**Example 7:**

N-oxide of N-piperidinyl-5-(4-chlorophenyl)-1-(2,4-dichlorophenyl)-4,5-dihydropyrazole-3-carboxamide

Under nitrogen gas as an inert atmosphere N-piperidinyl-5-(4-chlorophenyl)-1-(2,4-dichlorophenyl)-4,5-dihydropyrazole-3-carboxamide (0,15 g, 332 mmoles) was dissolved in 7 ml of dichloromethane. The resulting solution was ice-cooled to 0 °C and m-chloroperbenzoic acid (0,204 g, 0,83 mmoles) added in several portions. After stirring for 15 minutes a control via thin layer chromatography showed that no starting material was remaining. A saturated solution of sodium bicarbonate was then slowly added, the organic phase separated, washed with water, dried over sodium sulfate and filtered. The filtered solution was evaporated to dryness and the crude product was purified via column chromatography yielding 78 mg (50 % of theoretical yield) of the N-oxide of N-piperidinyl-5-(4-chlorophenyl)-1-(2,4-dichlorophenyl)-4,5-dihydropyrazole-3-carboxamide in form of a white solid having a melting point of 115-120 °C.

IR (KBr, $cm^{-1}$): 3202, 1678, 1654, 1474, 1309, 1107.

$^1H$-NMR ($CDCl_3$, δ): 1.6 (m, 2H), 1.8-2.0 (m, 4H), 2.55 (m, 2H), 3.3 (dd, J = 6.3 Hz and 18.2 Hz, 1 H), 3.7 (m, 3H), 5.8 (dd, J = 6.3 Hz and 12.5 Hz, 1 H), 7.0-7.3 (m, 7H), 8.5 (s, 1H.)

**Example 8:**

**5-(4-chlorophenyl)-1-(2,4-dichlorophenyl)-4,5-dihydro-pyrazole-3-carboxylic acid**

**a) 4-(4-chlorophenyl)-2-oxo-3-butenoic acid**

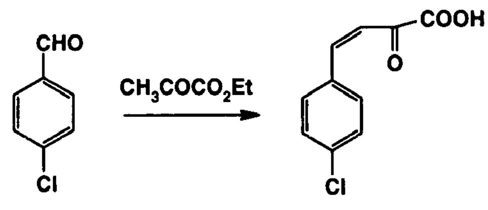


In a three neck flask p-chlorobenzaldehyde (13,3 g, 95 mmoles) and ethyl pyruvate (10 g, 86 mmoles) were dissolved in 150 ml of absolute ethanol.The solution was ice-cooled to 0°C and an aqueous solution of NaOH (3.8 g in 45 mL water) was added dropwise keeping the temperature below or equal to 10°C, whereby a yellow-orange colored precipitate was formed. The reaction mixture was stirred for 1 hour at 0°C and an additional 1.5 hours at room temperature (approximately 25 °C). Afterwards the reaction mixture was cooled down to approximately 5°C and the insoluble sodium salt of 4-(4-chlorophenyl)-2-oxo-3-butenoic acid was isolated by filtration.

The filtrate was left in the refrigerator overnight, whereby more precipitate is formed, which was filtered off, combined

with the first fraction of the salt and washed with diethyl ether. The sodium salt of 4-(4-chlorophenyl)-2-oxo-3-butenoic acid was then treated with a solution of 2N HCl, stirred for some minutes and solid 4-(4-chlorophenyl)-2-oxo-3-butenoic acid was separated via filtration and dried to give 12.7 g of the desired product (70% of theoretical yield).
IR (KBr, $cm^{-1}$): 3500-2500, 1719,3, 1686,5, 1603,4, 1587,8, 1081,9.
$^1H$ NMR($CDCl_3$, δ) : 7,4 (d, J=8,4Hz, 2H), 7,5 (d, J=16,1Hz, 1H), 7,6 (d, J=8,4Hz, 2H), 8,1 (d, J=16,1 Hz, 1 H).

**a2) 5-(4-chlorophenyl)-1-(2,4-dichlorophenyl)-4,5-dihydro-pyrazole-3-carboxylic acid**

In an alternative route instead of using ethylpyruvate the salt $CH_3$-C(O)-C(O)-$O^-Na^+$ (sodiumpyruvate) was used, dissolved ethanolic water.

**b) 5-(4-chlorophenyl)-1-(2,4-dichlorophenyl)-4,5-dihydro-pyrazole-3-carboxylic acid**

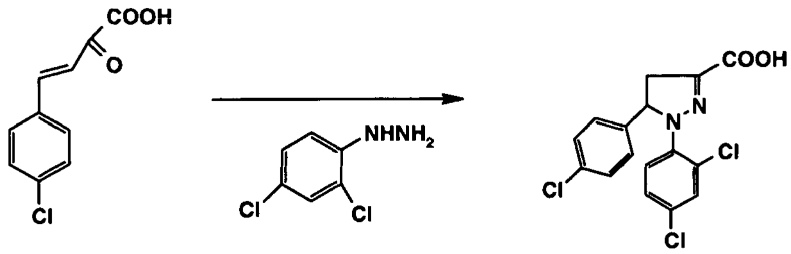

4-(4-chlorophenyl)-2-oxo-3-butenoic acid obtained according to step a) (12.6 g, 60 mmoles), 2,4-dichlorophenylhydrazine hydrochloride (12.8 g, 60 mmoles) and glacial acetic acid (200 mL) were mixed under a nitrogen atmosphere and heated to reflux for 4 hours, cooled down to room temperature (approximately 25 °C) and given into ice-water, whereby a sticky mass was obtained, which was extracted with methylene chloride. The combined methylene chloride fractions were washed with water, dried with sodium sulfate, filtered and evaporated to dryness to give a pale yellow solid (12.7 g, 57% of theoretical yield).
IR (KBr, $cm^{-1}$) : 3200-2200, 1668,4, 1458, 1251,4, 1104,8.
$^1H$ NMR ($CDCl_3$, δ) : 3,3 (dd, 1 H), 3,7 (dd, 1 H), 5,9 (dd, 1 H), 7,09-7,25 (m, 7H).

**Example 9:**

**N-piperidinyl-5-(4-chlorophenyl)-1-(2,4-dichlorophenyl)-4,5-dihydro-1H-pyrazole-3-carboxamide**

**(a) 5-(4-chlorophenyl)-1-(2,4-dichlorophenyl)-4,5-dihydro-pyrazole-3-carboxylic acid chloride**

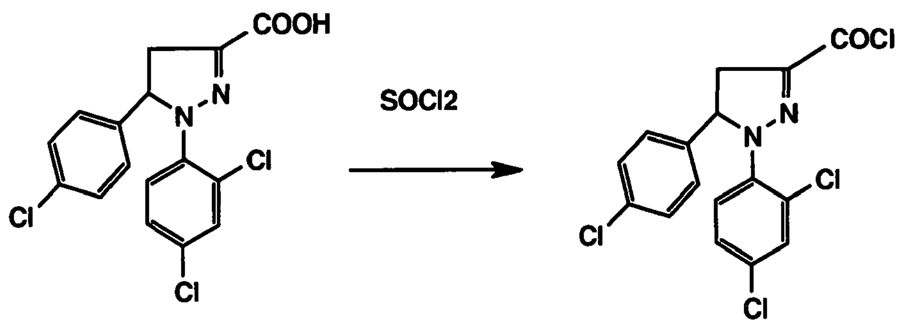


Under nitrogen atmosphere 5-(4-chlorophenyl)-1-(2,4-dichlorophenyl)-4,5-dihydro-pyrazole-3-carboxylic acid (2.5 g, 6.8 mmols) obtained according to Example 0 was dissolved in 4 mL of in thionyl chloride and heated to reflux for 2.5 hours. The excess thionyl chloride is removed from the reaction mixture under reduced pressure and the resulting crude residue (2.6 g) is used without any further purification.
IR (KBr, $cm^{-1}$) : 1732,3, 1700, 1533,3, 1478,1, 1212,9, 826,6.
Starting from this compound compounds according to general formulas II and III wherein $R^1$ is a linear or branched $C_{1-4}$-alkyl group can be prepared reacting this compound with the appropriate alkyl alcohol.

**(b) N-piperidinyl-5-(4-chlorophenyl)-1-(2,4-dichlorophenyl)-4,5-dihydropyrazole-3-carboxamide**

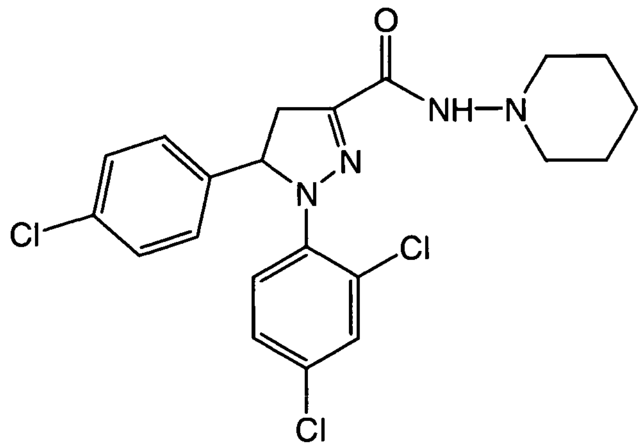

Under nitrogen atmosphere N-aminopiperidine (0.6 mL, 5.6 mmoles) and triethylamine (4 mL) were dissolved in methylene chloride (25 mL). The resulting mixture was ice-cooled down to 0°C and a solution of 5-(4-chlorophenyl)-1-(2,4-dichlorophenyl)-4,5-dihydro-pyrazole-3-carboxylic acid chloride obtained in step (c) in methylene chloride (15 mL) was added dropwise. The resulting reaction mixture was stirred at room temperature (approximately 25 °C) overnight. Afterwards the reaction mixture was washed with water, followed by a saturated aqueous solution of sodium bicarbonate, then again with water, dried over sodium sulfate, filtered and evaporated to dryness in a rotavapor. The resulting crude solid was crystallized from ethanol. The crystallized solid was removed via filtration and the mother liquors were concentrated to yield a second fraction of crystallized product. The two fractions were combined to give a total amount of 1.7 g (57% of theoretical yield) of N-piperidinyl-5-(4-chlorophenyl)-1-(2,4-dichlorophenyl)-4,5-dihydropyrazole-3-carboxamide having a melting point of 183-186°C.

IR (KBr, $cm^{-1}$) : 3222,9, 2934,9, 1647,4, 1474,7, 1268,3, 815,6.

$^1H$ NMR ($CDCl_3$, δ) : 1,4 (m, 2H), 1,7 (m, 4H), 2,8 (m, 4H), 3,3 (dd, J=6,1 y 18,3Hz, 1 H), 3,7 (dd, J=12,5 and 18,3 Hz, 1 H), 5,7 (dd, J=6,1 and 12,5 Hz, 1 H), 7,0-7,2 (m, 6H), 7,4 (s, 1H).

The compound according to the following example 9 has been prepared analogously to the process described in Example 10 in combination with Example 8.

**Example 10:**

**5-(4-Chloro-phenyl)-1-(2,4-dichloro-phenyl)-4,5-dihydro-1H-pyrazole-3-carboxylic acid-[1,2,4]triazol-4-yl amide**

Melting point: 134-138 ºC.

IR (KBr, $cm^{-1}$): 3448, 1686, 1477, 1243, 1091, 821.

$^1H$ NMR($CDCl_3$, δ): 3,1 (dd, J=6,2 and 17,9Hz, 1 H), 3,7 (dd, J=12,3 and 17,9Hz, 1 H), 5,9 (dd, J=6,2 and 12,3 Hz, 1 H), 7,2-7,5 (m, 7H), 8,7 (s, 2H), 12,0 (bs, 1H).

**Pharmacological Data:**

**I.In-vitro determination of affinity to $CB_1$/$CB_2$-Rezeptors**

The affinity of the inventive substituted pyrazoline compounds to $CB_1$/$CB_2$ receptors was determined as described above. Some of the values obtained are given in the following table I:

Table I:

| Compound according to Example | $CB_1$-Receptor Radiologand:[$^3$H]-CP55940 | | $CB_2$-Receptor Radiologand:[$^3$H]-CP55940 | |
|---|---|---|---|---|
| | % Inhibition $10^{-6}$ M | $K_i$(nM) | % Inhibition $10^{-6}$ M | $K_i$(nM) |
| 1 | 93 % | < 25 | 33 % | > 1000 |
| 5 | 79 % | 111 | 54 % | ≈ 1000 |

As can be seen from the values given in table 1 the inventive pyrazoline compounds are particularly suitable for regulating the $CB_1$-Receptor.

**II. In-vivo bioassay system for determination of cannabinoid activity**

The determinination of cannabinoid activity in-vivo was determined as described above. Some of the values obtained are given in the following table II:

Table II:

| Compound according to example: | dosis administered: 5 mg/kg i.v. Agonistic effect | | | | dosis administered 5 mg/kg i.v. prior to Win 55212-2 in a dose of 1,25mg/kg i.v. Antagonistic Effect | | | |
|---|---|---|---|---|---|---|---|---|
| | A | B | C | D | A | B. | C | D |
| 1 | 0 | 0 | 0 | 0 | 74 | 100 | 100 | 100 |
| 5 | 0 | 50 | 0 | 0 | 50 | 40 | 20 | 20 |

i.v. intravenous
A: Hot-Plate test
B: Hypothermia
C: Catalepsy
D: Sedation

As can be seen from the values given in table II the inventive pyrazoline compounds show an antagonistic effect.

**III. In-vivo testing for antiobesic activity**

The in-vivo testing for antiobesic activity was carried out as described above, whereby four different groups of 10 rats each were treated as follows:

Group I:

Group was treated with vehicle, namely arabic gum (5 wt.-%) in water.

Group II:

The second group of rats was treated with the inventive compound N-piperidinyl-5-(4-chlorophenyl)-1-(2,4-dichlorophenyl)-4,5-dihydropyrazole-3-carboxamide according to Example 1. Said compound was administered intraperitoneally to the rats over a period of 14 days in a daily dosis of (10 mg/kg body weight).

Group III:

The third group of rats was treated with Amphetamine, an active ingredient known to reduce appetite. Said compound was administered intraperitoneally to the rats over a period of 14 days in a daily dosis of (5 mg/kg body weight).

**IV. In vivo testing for antidepressant activity**

The in-vivo testing for antidepressant activity of the inventive pyrazoline compounds in the water despair test was carried out as described above. In particular, the compound according to example 1 displayed positive effects with respect to immobility time and struggling time.

**V. In-vivo testing for regulation of triglycerides in blood plasma**

The results obtained were the following :

| Group | Diet | Treatment | Triglicerides, whole blood levels (mg/dl) | Relative levels |
|---|---|---|---|---|
| I | Standard | - | 61 | 100% |
| II | High Fat | Vehicle 10 ml/kg/day po | 122.4 (*) | 200.6% |
| III | High Fat | Example 0 30 mg/kg/day po | 67.5 (N.S.) | 110.6% |
| (*) : p<0.05, Anova followed Bonferroni t-test, compared with Group I.<br>NS : Not significant diference, compared with Group I. | | | | |

The results showed that Group II mice receiving high fat diet had significantly higher triglicerides blood levels than the control Group I. But the administration of the compound according to Example 8 (Group III) improved the triglicerides blood levels, which were not different of the levels of the group I, which received standard diet.

## Claims

**1.** A pharmaceutical formulation comprising

(A) at least one substituted pyrazoline compound of general formula I

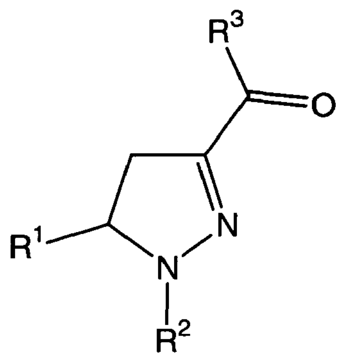

**I**

wherein

$R^1$ represents an optionally at least mono-substituted phenyl group;
$R^2$ represents an optionally at least mono-substituted phenyl group;
$R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or $R^3$ represents an optionally at least mono-substituted aryl or heteroaryl group, which may be condensed with an optionally at least mono-substituted mono- or polycyclic ring system, or $R^3$ represents an-$NR^4R^5$-moiety,
$R^4$ and $R^5$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted aryl or heteroaryl group, which may be condensed with an optionally at least mono-substituted mono- or polycyclic ring system and/or bonded via a linear or branched alkylene group; an—$SO_2$-$R^6$-moiety; or an -$NR^7R^8$-moiety, with the proviso that $R^4$ and $R^5$ do not identically represent

hydrogen;
$R^6$ represents a linear or branched, saturated or unsaturated, optionally at least mono-substituted aliphatic group; a saturated or unsaturated, optionally at least mono-substituted, optionally at least one heteroatom as ring member containing cycloaliphatic group, which may be condensed with a mono- or polycyclic ring-system; or an optionally at least mono-substituted aryl or heteroaryl group, which may be condensed with a mono- or polycyclic ring system and/or bonded via a linear or branched alkylene group;
$R^7$ and $R^8$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted aryl or heteroaryl group, which may be condensed with an optionally at least mono-substituted mono- or polycyclic ring system and/or bonded via a linear or branched alkylene group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof,
and/or
at least one substituted pyrazoline compound of general formula I',

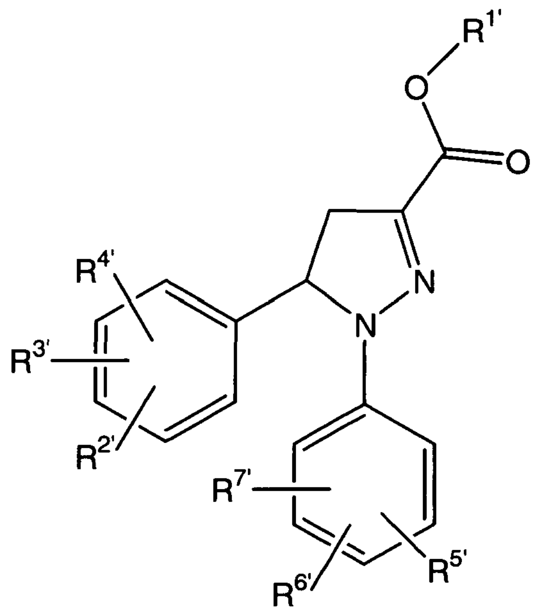

**I'**

wherein
$R^{1'}$ represents hydrogen or a linear or branched $C_{1-4}$-alkyl group,
$R^{2'}$, $R^{3'}$ and $R^{4'}$ independently of each other represent hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{8'}$, SH, $SR^{8'}$, $SOR^{8'}$, $SO_2R^{8'}$, $NH_2$, $NHR^{8'}$, $NR^{8'}R^{9'}$, -(C=O)-$NH_2$, -(C=O)-$NHR^{8'}$ or -(C=O)-$NR^{8'}R^{9'}$ whereby $R^{8'}$ and $R^{9'}$ for each substituent independently represent linear or branched
$C_{1-6}$ alkyl,
$R^{5'}$ and $R^{6'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10'}$, SH, $SR^{10'}$, $SOR^{10'}$, $NH_2$, $NHR^{10'}$, $NR^{10'}R^{11'}$, -(C=O)-$NH_2$, -(C=O)-$NHR^{10'}$ and -(C=O)-$NR^{10'}R^{11'}$, whereby $R^{10'}$ and optionally $R^{11'}$ for each substituent independently represent linear or branched $C_{1-6}$ alkyl;

$R^{7'}$ represents hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10'}$, SH, $SR^{10'}$, $SOR^{10'}$, $NH_2$, $NHR^{10'}$, $NR^{10'}R^{11'}$, -(C=O)-$NH_2$, - (C=O)-$NHR^{10'}$ and -(C=O)-$NR^{10'}R^{11'}$, whereby $R^{10'}$ and optionally $R^{11'}$ for each substituent independently represent linear or branched $C_{1-6}$ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof,
and

(B) nanoparticles,
wherein
component (A) is associated with component (B).

**2.** A pharmaceutical formulation according to claim 1, wherein said substituted pyrazoline compound of general formula I is selected from the group consisting of:

N-piperidinyl-5-(4-chloro-phenyl)-1-(2,4-dichlorophenyl)-4,5-dihydro-1H-pyrazol-3-carboxamide,
(rac)-piperidinyl-5-(4-chloro-phenyl)-1-(2,4-dichlorophenyl)-4,5-dihydro-1H-pyrazol-3-carboxamide,
(S)-N-piperidinyl-5-(4-chloro-phenyl)-1-(2,4-dichlorophenyl)-4,5-dihydro-1H-pyrazol-3-carboxamide, and
(R)-N-piperidinyl-5-(4-chloro-phenyl)-1-(2,4-dichlorophenyl)-4,5-dihydro-1H-pyrazol-3-carboxamide,
optionally in form of a corresponding N-oxide thereof, or a corresponding salt thereof, or a corresponding solvate thereof.

**3.** A pharmaceutical formulation according to claim 1, wherein said substituted pyrazoline compound of general formula I' is selected from the group consisting of:

5-(4-chloro-phenyl)-1-(2,4-dichlorophenyl)-4,5-dihydro-1H-pyrazol-3-carboxylic acid,
(rac)-5-(4-chloro-phenyl)-1-(2,4-dichlorophenyl)-4,5-dihydro-1H-pyrazol-3-carboxylic acid,
(S)-5-(4-chloro-phenyl)-1-(2,4-dichlorophenyl)-4,5-dihydro-1H-pyrazol-3-carboxylic acid, and
(R)-5-(4-chloro-phenyl)-1-(2,4-dichlorophenyl)-4,5-dihydro-1H-pyrazol-3-carboxylic acid, or a corresponding salt thereof, or a corresponding solvate thereof.

**4.** A pharmaceutical formulation according to one or more of claims 1 to 3, **characterized in that** the component (A) is covalently bound to component (B).

**5.** A pharmaceutical formulation according to one or more of claims 1 to 3, **characterized in that** the component (A) is bound to component (B) via van der Waals interaction, ionic interaction or hydrogen bonding.

**6.** A pharmaceutical formulation according to one or more of claims 1 to 5, **characterized in that** the component (A) forms a suspension or emulsion with component (B).

**7.** A pharmaceutical formulation according to one or more of claims 1 to 6, **characterized in that** the component (A) is enclosed in component (B).

**8.** A pharmaceutical formulation according to one or more of claims 1 to 7, **characterized in that** the component (A) is complexed, absorbed or adsorbed by component (B).

**9.** A pharmaceutical formulation according to one or more of claims 1 to 8, **characterized** that said formulation is administered orally or parenterally.

**10.** A pharmaceutical formulation according to claim 9, **characterized in that** the formulation is in form of a tablet, a chewable tablet, a coated tablet, a capsule, a drop, a dragee or a gum.

**11.** A pharmaceutical formulation according to claim 9, **characterized in that** the formulation is in form of a suppository, a fluid, a gel, an ointment, an emulsion, a foam, a balm, a lotion, a cream, a sleeve or a patch, optionally a transdermal patch.

**12.** A pharmaceutical formulation according to claim 9, **characterized in that** the formulation is an injectable solution.

**13.** A pharmaceutical formulation according to any of claims 1 to 12, **characterized in that** the formulation is an immediate-release formulation.

**14.** A pharmaceutical formulation according to any of claims 1 to 12, **characterized in that** the formulation is a controlled-release formulation.

**15.** A pharmaceutical formulation according to any of claims 1 to 12 and 14, **characterized in that** the formulation is a sustained-release formulation.

**16.** A pharmaceutical formulation according to any of claims 1 to 15 for use in medicine.

European Patent Office

# EUROPEAN SEARCH REPORT

Application Number
EP 05 38 4027

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 1 083 171 A (LABORATORIOS DEL DR. ESTEVE, S.A) 14 March 2001 (2001-03-14) * paragraphs [0060], [0061] * see especially compounds 34-42 | 1-16 | A61K31/4155 A61K31/496 A61K31/454 A61K9/51 |
| Y | EP 1 384 477 A (LABORATORIOS DEL DR. ESTEVE, S.A) 28 January 2004 (2004-01-28) * paragraph [0020]; claim 1 * see especially compounds 36, 39 and 42 | 1-16 | |
| Y,D | US 6 117 454 A (KREUTER ET AL) 12 September 2000 (2000-09-12) * column 7, line 33 - line 37 * * column 7, line 66 - column 8, line 40 * | 1-16 | |
| Y,D | CHANDRA SEKHARA RAO G ET AL: "NANOSUSPENSIONS AS THE MOST PROMISING APPROACH IN NANOPARTICULATE DRUG DELIVERY SYSTEMS" PHARMAZIE, DIE, PHARMAZEUTISCHER VERL., ESCHBORN, DE, vol. 59, no. 1, January 2004 (2004-01), pages 5-9, XP001186648 ISSN: 0031-7144 * page 59, right-hand column, paragraph 1 * | 1-16 | TECHNICAL FIELDS SEARCHED (IPC) A61K A61P |

The present search report has been drawn up for all claims

| Place of search | Date of completion of the search | Examiner |
|---|---|---|
| Munich | 18 November 2005 | Loher, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

3

EPO FORM 1503 03.82 (P04C01)

## ANNEX TO THE EUROPEAN SEARCH REPORT ON EUROPEAN PATENT APPLICATION NO. EP 05 38 4027

This annex lists the patent family members relating to the patent documents cited in the above-mentioned European search report.
The members are as contained in the European Patent Office EDP file on
The European Patent Office is in no way liable for these particulars which are merely given for the purpose of information.

18-11-2005

| Patent document cited in search report | Publication date | Patent family member(s) | Publication date |
|---|---|---|---|
| EP 1083171 A | 14-03-2001 | AT 265437 T | 15-05-2004 |
| | | AU 752001 B2 | 05-09-2002 |
| | | AU 3932999 A | 20-12-1999 |
| | | BG 105005 A | 31-08-2001 |
| | | BR 9910801 A | 27-11-2001 |
| | | CA 2333475 A1 | 09-12-1999 |
| | | CN 1307566 A | 08-08-2001 |
| | | DE 69916828 D1 | 03-06-2004 |
| | | DE 69916828 T2 | 14-04-2005 |
| | | DK 1083171 T3 | 09-08-2004 |
| | | ES 2137138 A1 | 01-12-1999 |
| | | WO 9962884 A1 | 09-12-1999 |
| | | ES 2221382 T3 | 16-12-2004 |
| | | HU 0102102 A2 | 28-03-2002 |
| | | JP 2002516908 T | 11-06-2002 |
| | | LT 2000108 A | 25-09-2001 |
| | | LV 12632 A | 20-03-2001 |
| | | LV 12632 B | 20-07-2001 |
| | | NO 20006029 A | 26-01-2001 |
| | | NZ 508990 A | 20-12-2002 |
| | | PL 344412 A1 | 05-11-2001 |
| | | PT 1083171 T | 30-09-2004 |
| | | RU 2233272 C2 | 27-07-2004 |
| | | SI 20580 A | 31-12-2001 |
| | | SK 18072000 A3 | 10-07-2001 |
| | | TW 572898 B | 21-01-2004 |
| | | UA 61137 C2 | 15-03-2001 |
| | | US 6353117 B1 | 05-03-2002 |
| | | ZA 200007638 A | 13-11-2001 |
| EP 1384477 A | 28-01-2004 | BR 0208805 A | 13-07-2004 |
| | | CA 2442974 A1 | 17-10-2002 |
| | | CN 1509171 A | 30-06-2004 |
| | | WO 02080909 A1 | 17-10-2002 |
| | | ES 2174757 A1 | 01-11-2002 |
| | | HU 0400918 A2 | 28-07-2004 |
| | | JP 2004525166 T | 19-08-2004 |
| | | MA 27019 A1 | 20-12-2004 |
| | | NO 20034470 A | 05-12-2003 |
| | | PL 365220 A1 | 27-12-2004 |
| | | US 2004034082 A1 | 19-02-2004 |
| | | ZA 200308626 A | 05-11-2004 |
| US 6117454 A | 12-09-2000 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6117454 A


### Non-patent literature cited in the description


- **HOLLISTER.** *Pharm. Rev.,* 1986, vol. 38, 1-20
- **RENY ; SINGHA.** *Prog. Drug. Res.,* 1991, vol. 36, 71-114
- **CONSROE ; SANDYK.** Marijuana/Cannabinoids, Neurobiology and Neurophysiology. CRC Press, 1992
- *Pharmazie,* 2004, vol. 59, 5-9
- *J Pharm Pharmaceut Sci,* 2000, vol. 3, 234-258
- *Expert Rev Vaccines,* 2003, vol. 2, 753-761
- *Adv Drug Delivery Rev,* 2002, vol. 54, 131-155
- **BANKS, W. A. ; KASTIN, A. J. ; BARRERA.** Delivering peptides to the central nervous system: Dilemmas and strategies. *Pharm. Res.,* 1991, vol. 8, 1345-1350
- *Synthetic communications,* 1996, vol. 26 (11), 2229-33
- *Synlett,* 2001, 147-149
- **PASCUAL, A.** *J. Prakt Chem.,* 1999, vol. 341 (7), 695-700
- **LIN, S. et al.** *Heterocycles,* 2001, vol. 55 (2), 265-277
- **RAO, P. et al.** *J. Org. Chem.,* 2000, vol. 65 (22), 7323-7344
- **PEARSON D.E ; BUEHLER, C.A.** *Synthesis,* 1972, 533-542
- **RUTH A. ROSS ; HEATHER C. BROCKIE et al.** Agonist-inverse agonist characterization at CB1 and CB2 cannabinoid receptors of L-759633, L759656 and AM630. *British Journal of Pharmacology,* 1999, vol. 126, 665-672
- **A. C. HOWLETT et al.** International Union of Pharmacology XXVII. Classification of Cannabinoid Receptors. *Pharmacol Rev,* 2002, vol. 54, 161-202
- **DAVID R. COMPTON et al.** In-vivo Characterization of a Specific Cannabinoid Receptor Antagonist (SR141716A) :Inhibition of Tetrahydrocannbinol- induced Responses and Apparent Agonist Activity. *J. Pharmacol. Exp. Ther.,* 1996, vol. 277 (2), 586-594
- **WOOLFE D. et al.** The evaluation of analgesic action of pethidine hydrochloride (Demerol. *J. Pharmacol. Exp. Ther.,* 1944, vol. 80, 300-307
- **DESMET L. K. C. et al.** Anticonvulsive properties of Cinarizine and Flunarizine in Rats and Mice. *Arzneim. -Forsch. (Frug Res,* 1975, vol. 25 (9
- **DAVID R. COMPTON et al.** In-vivo Characterization of a Specific Cannabinoid Receptor Antagonist (SR141716A) Inhibition of Tetrahydrocannbinol- induced Responses and Apparent Agonist Activity. *J. Pharmacol Exp Ther.,* 1996, vol. 277 (2), 586-594
- **ALPERMANN H. G. et al.** Pharmacological effets of Hoe 249: A new potential antidepressant. *Drugs Dev. Res.,* 1992, vol. 25, 267-282
- **G. COLOMBO et al.** Appetite Suppression and Weight Loss after the Cannabinoid Antagonist SR 141716. *Life Sciences,* 1998, vol. 63 (8), 113-117